# EUROPEAN PATENT APPLICATION

(11) **EP 4 324 463 A1**
(43) Date of publication of application: **21.02.2024**
(21) Application number: 22795016.9
(22) Date of filing: 29.04.2022
(51) Int. Cl.: A61K 31/415, A61K 9/00, A61P 27/10, A61P 27/06, A61P 27/12, A61P 27/02

(54) **METHOD AND PHARMACEUTICAL COMPOSITION FOR TREATING MYOPIA**

(30) Priority: 30.04.2021 CN 202110485864
(71) Applicant: Grand Pharma (China) Co., Ltd., Wuhan, Hubei 430030 (CN)
(72) Inventor: ZHOU, Xiangtian, Wenzhou, Zhejiang 325027 (CN); PAN, Miaozhen, Wenzhou, Zhejiang 325027 (CN); QU, Jia, Wenzhou, Zhejiang 325027 (CN); ZHENG, Qinyuan, Wenzhou, Zhejiang 325027 (CN); WU, Hao, Wenzhou, Zhejiang 325027 (CN)
(74) Representative: DehnsGermany Partnerschaft von Patentanwälten
(86) International application number: PCT/CN2022/090230
(87) International publication number: WO 2022/228546

(57) **Abstract**

The present application relates to a method and pharmaceutical composition for treating myopia. The pharmaceutical composition or the method of the present application can effectively prevent and control myopia, is safe and has no obvious side effect, and has a good clinical application prospect.

## Description

### FIELD

This application relates to methods and drugs for treatmenting, preventing, or controling myopia and its related symptoms.

### BACKGROUND

In 350 BC, Aristotle first used the term "myopia" (Paulus T V M de Jong, Br J Ophthalmol. 2018 Aug; 102 (8): 1021-102) to refer to a phenomenon of ametropia, which is currently one of the most serious public health problems in the world. Early literature indicated that the global patients of myopia in 2010 was 1.84 billion in 2010, accounting for 27% of the total world population at that time, including 170 million people with high myopia, accounting for 2.8%. Especially in East Asia, such as China, Japan, Korea, and Singapore, the prevalence of myopia was close to 50%, much higher than in Australia, Europe, and America. It is estimated that the global prevalence of myopia will reach 50% or more by 2050 (Brien A Holden, Ophthalmology. 2016 May;123(5):1036-42). The incidence of myopia is particularly severe in children and adolescents aged 6 to 18 in China. In 2016, an epidemiological survey of 57904 samples showed that the prevalence of myopia among primary and middle school students in six provinces and cities in North China, East China, South China, Southwest and Northwest China was 55.7%. Among them, the prevalence of myopia in the 6-8-years-old group, 10-12-years-old group, 13-15-years-old group, and 16-18-years-old group were 35.8%, 58.9%, 73.4%, and 81.2%, respectively (Zhou Jia, Ma Yinghua and Ma Jun et al. "Prevalence of myopia and influencing factors among primary and middle school students in 6 provinces of China". Chinese Journal of Epidemiology. 2016; 37:29-34.). Epidemiological data show that the global prevalence of myopia and high myopia is constantly increasing, and significant changes in social, lifestyle, and environmental factors are one of the important reasons that affect the increasing prevalence, low aging and severity of myopia (Susan Vitale, Arch Ophthalmol. 2009 Dec;127(12):1632-9). This trend is not significantly improved over a short period of time.

There are many disadvantages resulted from myopia. In addition to blurring of distance vision, myopia, especially high myopia, may also cause severe complications such as glaucoma, cataract, retinal detachment, retinal tear, posterior scleral staphyloma, macular hemorrhage or myopic macular degeneration, choroidal neovascularization and so on, impairing vision-related quality of life, increasing the difficulty of vision-related work and resulting in vision impairment and even blindness (Chen-Wei Pan, Ophthalmic Physiol Opt. 2012 Jan; 32(1):3-16, and Seang-Mei Saw, Ophthalmic Physiol Opt. 2005 Sep;25(5):381-91.).

### SUMMARY

In view of the above technical problems, the present application provides use of bendazac lysine or bendazac, or an optical isomer thereof, a racemate thereof, a solvate thereof, a pharmaceutically acceptable salt thereof, a prodrug thereof, a metabolite thereof, an analog thereof or a derivative thereof, a crystalline compound thereof, or a combination of these substances, in one or at least two of the following:
(a) preventing and/or treating myopia and myopia-related symptoms;
(b) delaying, reducing, or treating an abnormal development of eyeball associated with ametropia;
(c) enabling an individual to obtain clearer distance vision without using or replacing lenses (such as myopia frame glasses or OK lenses) or relying on other vision correction means (such as refractive surgery);
(d) controlling, inhibiting, delaying, or slowing down the progress (speed) of diopter (continuously) becoming negative in an individual with myopia or a tendency to develop myopia;
(e) preventing and/or treating myopia and related symptoms thereof in combination with surgery (such as refractive correction surgery, myopia corneal laser surgery, lens surgery) or other vision correction means (such as corneal contact lenses);
(f) preventing and/or treating myopia and myopia-related symptoms in combination with one or more other drugs;
(g) reducing a distance between a retina and a lens, preferably reducing the distance between the retina and the lens in an individual with myopia or a tendency to develop myopia;
(h) reducing a degree of myopia, or treating myopia, or treating nearsightedness, or controlling a progression of myopia, or correcting myopia, or alleviating myopia, or preventing myopia in adolescents;
(i) inhibiting or treating myopia caused by lens lesions; and
(j) manufacturing a pharmaceutical composition, preparation, or device to fulfill at least one of the uses of (a) to (i) above.

In some embodiments, bendazac lysine or bendazac, or the optical isomer thereof, the racemate thereof, the solvate thereof, the pharmaceutically acceptable salt thereof, the prodrug thereof, the metabolite thereof, the analog thereof or the derivative thereof, the crystalline compound thereof, or the combination of these substances acts as a sole active ingredient or as a main active ingredient.

In some embodiments, a content of the sole active ingredient or the main active ingredient accounts for 50% or more, 60% or more, 70% or more, 80% or more, 90% or more, or 100% of total active ingredients, in which the percentage is a mass ratio or a molar ratio.

In some embodiments, these substances or the combination thereof and the one or more other drugs are formulated or designed in a manner of continuous administration, simultaneous administration, sequential administration, alternating administration, interval administration, or separate administration.

In some embodiments, the abnormal development of eyeball associated with ametropia is developed during the juvenile phase of an individual (such as 2-28 years of age in humans), and is mainly induced by environmental factors or mainly caused by human factors (such as long-term near-distance reading, frequent use of electronic screens, persistent nearsightedness and lack of chance for farsightedness, improper use of refractive corrective glasses, side effects of drugs, obesity, trauma, poor lighting in the learning environment, lack of outdoor exercises), while genetic factors are secondary factors, concomitant factors, synergistic factors, or the abnormal development is a refractive dysplasia completely independent of genetic factors, mainly characterized in that in a state of accommodative relaxation, a focus falls in front of the retina after parallel light rays are refracted through a refractive system of eyes.

In some embodiments, systemic administration (such as oral administration, intravenous drip), topical administration (such as eye drop, intravitreal injection, skin cream or ointment application), parenteral administration (such as via mucosal administration, transdermal administration, microneedle administration), non-invasive administration (such as by applying an ophthalmic ointment to the cornea or squeezing into a sac formed by stretching of the lower eyelid), or non-invasive administration (such as using an ophthalmic spray) is used.

In some embodiments, the skin cream or ointment application is a application with 3% skin cream or ophthalmic ointment.

In some embodiments, the administrations (such as eye drop, oral administration) are used simultaneously, in combination, alternately, at intervals, separately, or one of them selected for use.

In some embodiments, wherein a preparation for topical administration includes but is not limited to aqueous, oily, or suspension preparation, that can incorporate pharmacologically and/or physiologically active ingredients such as a mydriatic component, decongestant component, eye muscle (such as ciliary muscle) regulating component, anti-inflammatory component, astringent component, antihistamine component, antiallergic component, hepatoprotective (avoiding or attenuating hepatotoxicity) component, blood-retinal barrier enhancing component (making it more difficult for compounds to penetrate through the physiological barrier), vitamin, amino acid, antibacterial agent component, saccharide, polymer or derivative thereof, cellulose or derivative thereof, local anesthetic component, glaucoma treatment component, cataract treatment component, and the like.

In some embodiments, a concentration or a ratio of these substances or the combination thereof in the pharmaceutical composition, preparation, or device is at least not less than 0.01%, preferably 0.01% to 0.8%, preferably 0.05% to 0.5%, more preferably 0.1%; or the concentration or the ratio of these substances or the combination thereof is less than 0.01%, in which the percentage being expressed as mass/volume concentration (ratio) or mass ratio or molar (number) ratio.

In some embodiments, the pharmaceutical composition or preparation can be an injection, tablet, lyophilized powder injection, capsule, effervescent tablet, chewable tablet, buccal tablet (lozenge), granule, ointment, syrup, oral liquid, aerosol, nasal drops, external preparation, oral preparation and the like; preferably ophthalmic dosage form, including but not limited to eyedrops (eye drops), eye ointment, eye spray, implant, ophthalmic gel, ophthalmic patch, ophthalmic microsphere, ophthalmic sustained-release preparation, periocular injection or intraocular injection; or a free solution, oil-water mixture, suspension, liniment, lotion, cream, drop, electuary, spray, ointment, patch, paste, pill, suppository or emulsion.

In some embodiments, the individual with myopia or a tendency to develop myopia is a human being, and can be a child, adolescent, middle-aged person or elderly person, preferably a person aged 3 to 26, more preferably a person aged 6 to 18; or an adult or minor, preferably those whose eyes (eyeballs) are still in the growth and development stage; or a school-age people, preferably students in grades 1 to 12.

In some embodiments, the myopia is refractive myopia or axial myopia; congenital myopia (birth or preschool myopia), early-onset myopia (under 14 years old), delayed myopia (16 to 18 years old), late-onset myopia (after adulthood); low myopia (mild myopia), moderate myopia, high myopia (severe myopia); pseudo-myopia, true-myopia, semi-true and semi-pseudo (mixed) myopia; myopia in children and/or adolescents (preferably the age of the population is 3 to 26, more preferably the age of the population is 6 to 18), myopia in minors, myopia in adolescents, myopia in adults, myopia in the elderly; simple myopia, pathological myopia; axial simple myopia, simple axial myopia; axial myopia in children and/or adolescents (preferably the age of the population is 3 to 26, more preferably the age of the population is 6 to 18); axial myopia in school age and pre-school population; primary myopia, secondary myopia; primary myopia in children and/or adolescents (preferably the age of the population is 3 to 26, more preferably the age of the population is 6 to 18); progressive myopia in children and/or adolescents (preferably the age of the population is 3 to 26, more preferably the age of the population is 6 to 18); curvature myopia, index myopia, positional myopia, bending myopia; myopia caused by long-term short-distance use of eye(s), myopia and pseudo-myopia caused by asthenopia, negative diopter caused by adverse drug reactions, nearsightedness, myopia caused by reading, myopia caused by the use of electronic products such as mobile phones, myopia caused by mismatch of refractive media (components), refractive myopia, myopia caused by abnormal refractive development, myopia caused by excessive eyeball growth, myopia caused by unhygienic use of eyes, myopia caused by imaging focus of distant object falling in front of the retina caused by various reasons, myopia caused by poor or ineffective treatment of atropine, myopia caused by insufficient outdoor exercises, and accommodative tension myopia, childhood myopia, or myopia dominated by environmental factors.

In some embodiments, the myopia-related symptoms include complications resulting from myopia, such as complications resulting from high myopia, muscae volitantes, glaucoma, posterior scleral staphyloma, retinal detachment, retinal tear, amblyopia, macular hemorrhage, choroidal neovascularization, choroidal atrophy, macular degeneration or maculopathy, visual field loss, progressive or sudden decrease in vision (especially near vision), ocular soreness and/or pain, night blindness, astigmatism, anisometropia, blindness, vitreous liquefaction, vitreous opacity, strabismus, frequent blinking, frequent eye rubbing, anisometropia, blurred vision when looking at distant objects, squinting or partially closing eyelids to see distant objects clearly, headache caused by asthenopia, difficulty in vision when driving, especially at night (night myopia), retinal atrophy and degeneration (bleeding and tears), subretinal neovascularization, and eyeball atrophy.

In some embodiments, the pharmaceutical composition, preparation, or device further includes a medicinal preparation or drug comprising, but not limited to, a drug for treating myopia (e.g. atropine, dibazole, pirenzepine, muscarinic antagonist, 7-methylxanthine (7MX), aminobenzylamine, indoleamine, timolol maleate, epinephrine, pirenzepine, pyrazine, pybenpine, pibenpine, pyenzepine, methylamine, chlorisondamine, acetylcholinesterase inhibitor, dopamine agonist, gamma-aminobutyric acid, naloxone, glucagon, retinoic acid, etc.), M receptor blocker (such as a blocker or antagonist or inhibitor for the M3 receptor), bendazac or various salt forms thereof, bendazac lysine or various salt forms thereof, polyunsaturated fatty acid (such as DHA, EPA), salidroside, formononetin, prazosin, homatropine, anisodamine (racemic), tropicamide, nicotinic acid, piracetam, salvia miltiorrhiza extract, safflower extract, fish oil, bear bile extract, vitamin, ATP, non-selective adenylate antagonist, vasodilator, mydriatic, smooth muscle relaxant, vasospasm preventing drug, collagen metabolism regulating drug, anti-allergic drug, anti-inflammatory drug, liver-protecting drug, therapeutic component for ophthalmic disease, topical ophthalmic anesthetic, or ophthalmic preparation.

In some embodiments, the one or more other drugs comprise, but are not limited to, a drug for treating myopia (e.g. atropine, dibazole, pirenzepine, muscarinic antagonist, 7-methylxanthine (7MX), pirenzepine, aminobenzylamine, indoleamine, timolol maleate, epinephrine, pyrazine, pybenpine, pibenpine pyenzepine, methylamine, chlorisondamine, acetylcholinesterase inhibitor, dopamine agonist, gamma-aminobutyric acid, naloxone, glucagon, retinoic acid, etc.), M receptor blocker (such as a blocker or antagonist or inhibitor for the M3 receptor), bendazac or various salt forms thereof, bendazac lysine or various salt forms thereof, polyunsaturated fatty acid (such as DHA, EPA), salidroside, formononetin, prazosin, homatropine, anisodamine (racemic), tropicamide, nicotinic acid, piracetam, salvia miltiorrhiza extract, safflower extract, fish oil, bear bile extract, vitamin, ATP, non-selective adenylate antagonist, vasodilator, smooth muscle relaxant, vasospasm preventing drug, collagen metabolism regulating drug, anti-allergic drug, anti-inflammatory drug, liver-protecting drug, therapeutic component for ophthalmic disease, topical ophthalmic anesthetic, mydriatic, or ophthalmic preparation or drug.

In some embodiments, the preparation can also be an oral product such as a health product, food product, dietary supplement, nutritional product, drink, or a cosmetic product; wherein the cosmetic product can be one or more of a free solution, oil-water mixture, suspension, liniment, lotion, spray, cream, drop, electuary, ointment, paste, pill, suppository, emulsion, and patch.

In some embodiments, the device is an instrument, apparatus, consumable, system, medical device, health care product or product for changing the appearance of the eye, capable of releasing a drug or having a drug delivery function or a potential drug delivery capability, such as corneal contact lens(es), glasses, intraocular lens, suture, OK lens(es) cleaning (maintenance) system, eye patch, eyesight improving patch, cosmetic lens(es), microneedle, eye spray system, eye massager (myopia massager), eye fumigator, ocular surface drug delivery device, intraocular drug delivery device, fundus drug delivery device, implanted pump, wearable equipment, acupoint massage instrument, eye relaxation equipment, myopia treatment instrument, or drug-device combination for myopia prevention and control.

### BRIEF DESCRIPTION OF DRAWINGS

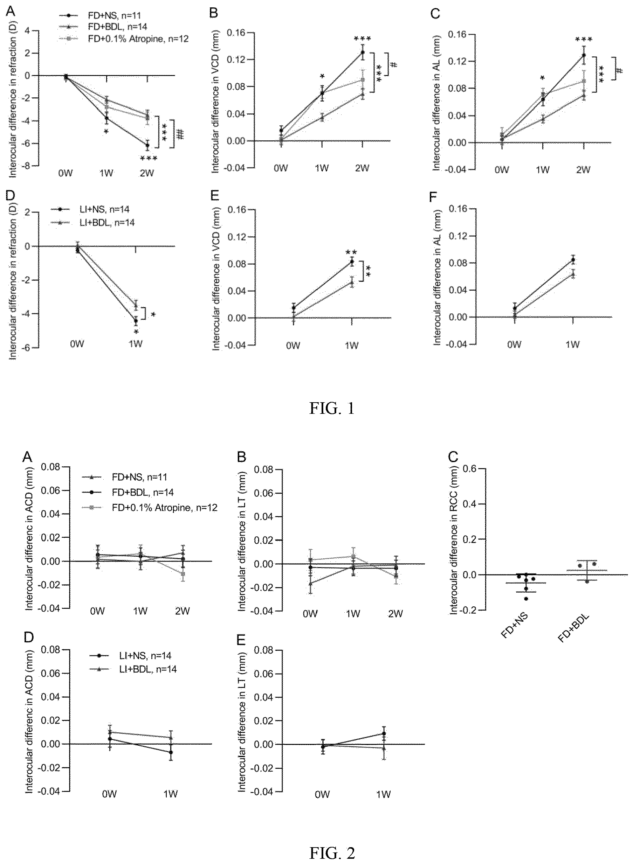

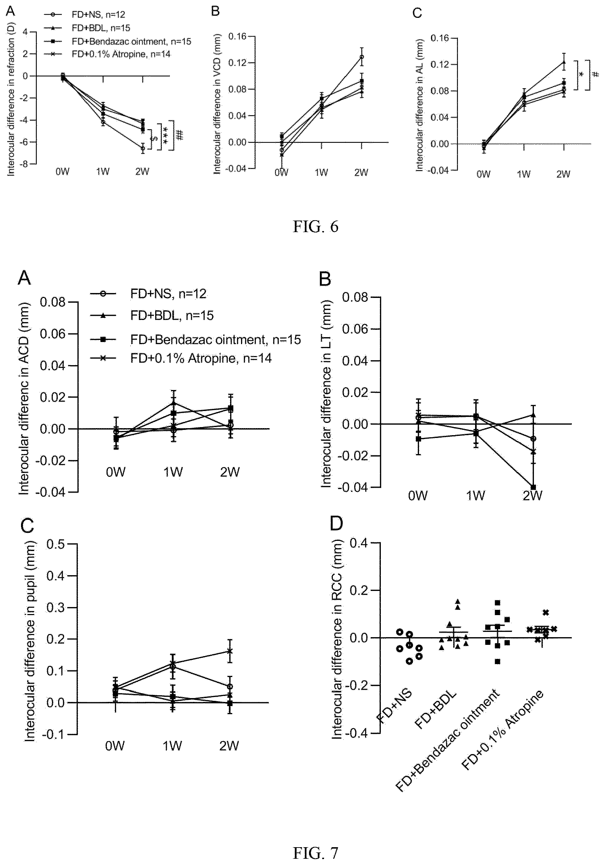

FIG. 1 shows bendazac lysine controls myopia progression.
FIG. 2 shows safety of treatment with bendazac lysine eye drop.
FIG. 3 shows prevention and control of myopia with different concentrations of bendazac lysine eye drops.
FIG. 4 shows safety of treatment with different concentrations of bendazac lysine.
FIG. 5 shows bendazac lysine increases choroidal thickness in individuals with myopia.
FIG. 6 shows therapeutic effects of eyedrop administration of bendazac lysine eye drops and application of bendazac ointment to eye(s).
FIG. 7 shows safety of eyedrop administration of bendazac lysine eye drops and application of bendazac ointment to eye(s).
   Among them, in FIGs. 1-5, * represents the statistical difference between bendazac lysine (bendazac) administration group and negative control group; # represents the statistical difference between atropine administration group and negative control group. * represents p <0.05; ** represents p <0.01; *** represents p <0.001; # represents p <0.05; ## represents p <0.01; ### represents p <0.001. In FIGs. 6 and 7, * represents the difference between bendazac lysine eye drops group and normal saline group; # represents the statistical difference between atropine administration group and normal saline group; $ represents the difference between bendazac ointment group and normal saline group. * represents p <0.05; *** represents p <0.001; # represents p <0.05; ## represents p <0.01; $ represents p<0.05.

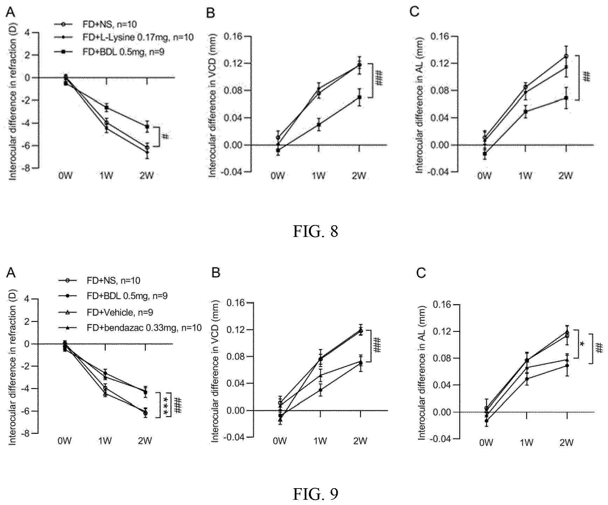

FIG. 8 shows lysine alone has no effect of treating myopia; # represents the difference between the BDL group and the saline vehicle group; # represent p <0.05; ## represent p <0.01; ## represent p<0.001.
FIG. 9 shows bendazac eye drops alone have the same therapeutic effect as bendazac lysine eye drops in treating myopia. Among them, # represents the difference between the BDL group and the saline vehicle group; * represents the difference between bendazac group and the vehicle solvent group. * represents p <0.05; *** represents p <0.001; ## represents p <0.01; and ### represents p <0.001.

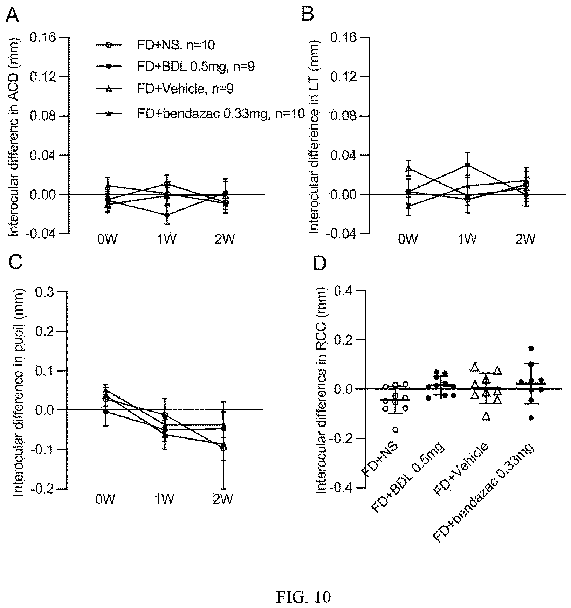

FIG. 10 shows safety of treatment with bendazac alone.

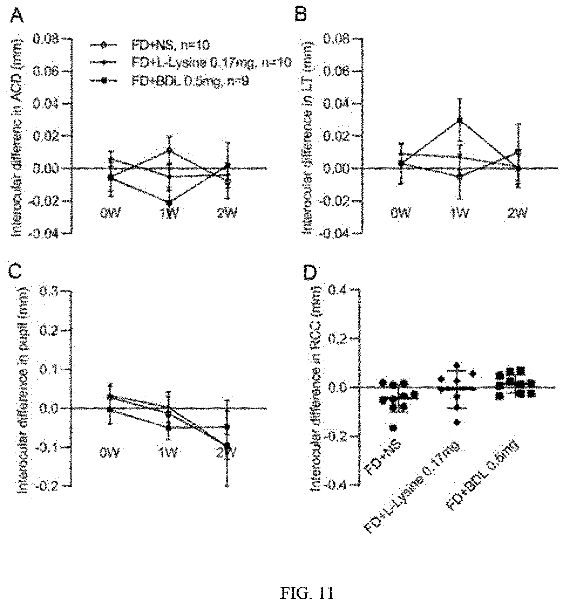

FIG. 11 shows safety of treatment with lysine alone.

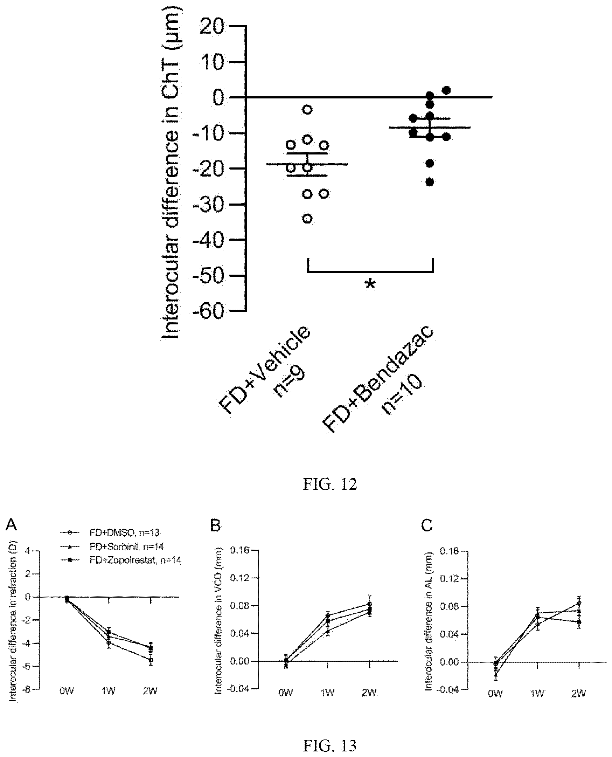

FIG. 12 shows bendazac inhibits the reduction of choroidal thickness in individuals with myopia, with * representing p <0.05.
FIG. 13 shows Sorbinil and Zopolrestat have no effect of treating myopia.

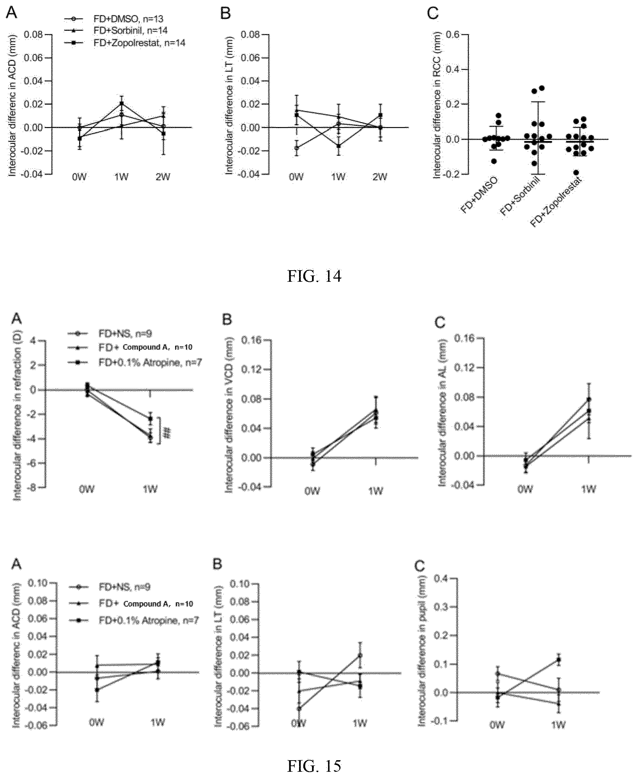

FIG. 14 shows safety evaluation of administration of aldose reductase inhibitor(s).
FIG. 15 shows m-hydroxymethylaniline has no effect of treating myopia. # represents the statistical difference between atropine administration group and negative control group. ## represents p<0.01.

### DESCRIPTION OF EMBODIMENTS

The following will provide a detailed description of the embodiments of the technical solution of this application in conjunction with the accompanying drawings. The following embodiments are provided only to more clearly illustrate the technical solution of the present application, and are therefore used as examples only, without limiting the scope of the present application.

The experimental methods used in the following embodiments are conventional methods unless otherwise specified. Materials, reagents, and the like used in the following embodiments are commercially available unless otherwise specified.

Myopia is the most common ametropia and refers to a refractive condition in which the focus falls in front of the retina after parallel light rays are refracted through the refractive system of the eyes in a state of accommodative relaxation. There are four common categories of myopia: (1) according to the diopter size, it can be divided into mild (low) myopia, moderate myopia, and high (severe) myopia; (2) according to whether the refractive component is abnormal, it can be divided into refractive myopia and axial myopia; (3) according to whether there are pathological changes, it can be divided into pathological myopia and simple myopia; (4) according to the classifications of the causes, it can be divided into primary myopia and concurrent/secondary myopia. Regardless of the onset and development of myopia, the mismatch between the respective components of the refractive system (i.e. the refractive medium, including cornea, lens and vitreous, etc.) is the key to the formation and severity of myopia. Taking refractive myopia as an example, under the premise that other refractive components are normal, if corneal curvature of a individual is abnormal, the focus of the parallel light rays after passing through the cornea will deviate from the original position on the retina, thereby causing diopter change, commonly the myopia caused by keratoconus. For another example, if the diopter of the lens changes due to some pathological changes, the projection position of parallel light rays on the retina will also be directly affected. In this case, if the focus of parallel light rays fall in front of the retina, it is called myopia caused by lens lesions. The mismatch of refractive components also includes a retroposition of the retina due to an oversized vitreous chamber, resulting in the intra-ocular imaging focus of a distant object falls in front of the retina, which is manifested as negative diopter change. It can be seen that the above-mentioned myopia classification method will be further subdivided according to specific situations in actual clinical practice. Nevertheless, diopter is generally recognized as the only indicator for determining all types of myopia, measuring their severity, and evaluating the effectiveness of treating myopia. According to the "Technical Guidelines for Clinical Research on Drugs for Controlling Myopia Progress" and the consensus of the academic community, the most common type of myopia in children and adolescents (especially those aged 6-18) is axial simple myopia (Paul N Baird, Nat Rev Dis Primers. 2020 Dec 17;6(1):99; A J Adams, Am J Optom Physiol Opt. 1987 Feb; 64(2): 150-2; and Seang-Mei Saw, Ophthalmic Physiol Opt. 2005 Sep;25(5):381-91.), wherein the main site of elongation of axial length is in the posterior pole of the eyeball. Consistent with myopia in human, it has been found the axial lengthening, scleral thinning, and scleral collagen thinning have also been occurred in long-term experimental myopia models of mammals (e.g. tree mice, marmosets, guinea pigs) (Neville A McBrien, Prog Retin Eye Res. 2003 May;22(3):307-38.). In the preclinical development phase of practical drugs, researchers almost always use two classical disease models for myopia, i.e., form-deprivation (FD) or lens induced (LI) models, to evaluate the efficacy of preventing and controlling myopia (D A Goss, Am J Optom Physiol Opt. 1981 Oct;58(10):859-69; and Hao Wu, Proc Natl Acad Sci U S A. 2018 Jul 24;115(30):E7091-E7100; and Sen Zhang, Invest Ophthalmol Vis Sci. 2019 Jul 1;60(8):3074-3083.), and compounds with both therapeutic and prophylactic effects on these two models of myopia are considered to be the most likely to be drugs.

The pathogenesis of myopia remains not fully understood. At present, environmental factors are considered to be the main cause of myopia, affecting the prevalence and severity of myopia, while cases of myopia caused by genetics is relatively rare. Environmental factors include excessive accommodation, hyperopic defocus of peripheral retinal, lighting illumination (abnormal lighting), form deprivation, etc. The specific mechanism of myopia may be that after the retina recognizes the near vision information that induces myopia, these signals are transmitted to the sclera through the choroid, resulting in changes in the extracellular matrix components of the sclera, ultimately inducing a decrease in diopter until the diopter becomes negative, and resulting in myopia. This can also be simply summarized as optical defocus triggers a defocus-specific signals, thereby regulating the refractive development of the eye (Wen-Yi Wang, Biomed Pharmacother. 2021 Jan;133:111092; Tatiana V Tkatchenko, Trends Pharmacol Sci. 2019 Nov;40(11):833-852; and David Troilo, Invest Ophthalmol Vis Sci. 2019 Feb 28;60(3): M31-M88.). In a normally developing individual, the size of the eye also grows along with other parts of the body. Humans, like other mammals, show hyperopia from birth to childhood. In the subsequent growth stage, with the further development of the refractive components in the eyeball and a modest elongation of the axis length of the eye, the imaging focus of the parallel light rays will overlap with the retina (i.e. the focus falls on the retina) and it turns to emmetropia. If there is a long-term continuous near vision information during this development, a series of diseases caused by mismatch of refractive components occur, including an excessive elongation of the axis length of the eye, causing the imaging focus of the parallel light rays to fall in front of the retina, resulting in myopia. Therefore, how to ensure that the refractive components match each other in the process of eyeball development and prevent eyeball overgrowth is the key to the prevention and control of myopia. Previous studies have found that abnormal refractive development in individuals with myopia is related to scleral remodeling, loss of scleral tissue due to reduced synthesis of connective tissue, and increased degradation of collagen type 1 (COL1), where dopamine, insulin, and nitric oxide may be involved.

At present, frame glasses are the main way to correct myopia in children and adolescents, and adult patients can use laser surgery. Although myopia can be corrected in most cases by eyeglasses, contact lenses, or refractive surgery, its progress cannot be delayed. Any type of myopia that develops to high myopia would be a particularly dangerous vision problem because of the high risk of retinal, choroidal and scleral complications. Therefore, correction of myopia cannot be simply understood as treatment of myopia. Clinically, the treatment of myopia is mainly to inhibit or slow down the progress of myopia, involving both optical and pharmacological approaches. Orthokeratology can delay the progress of myopia in children and adolescents, but the efficacy of this treatment varies widely among individuals and requires close assistance from a professional optometrists (Jinhai Huang, Ophthalmology.2016 Apr;123(4):697-708). The pharmacological options for myopia control are very limited (Tatiana V Tkatchenko, Trends Pharmacol Sci. 2019 Nov;40(11):833-852.), e.g. atropine eye drops have shown efficacy in the treatment of myopia in many studies, but it was observed clinically that the degree of myopia rebounded after drug withdrawal, and the use process was accompanied by serious side effects such as mydriasis and photophobia (Prema Ganesan, Expert Rev Ophthalmol. 2010 Dec 1;5(6):759-787.), which has not been approved for use by National Medical Products Administration in China. 7-methylxanthine is another drug under investigation for controlling myopia, and its safety and effectiveness need more data to support (Klaus Trier, J Ocul Biol Dis Infor. 2008 Dec;1(2-4):85-93; and Tatiana V Tkatchenko, Trends Pharmacol Sci. 2019 Nov;40(11):833-852.). Therefore, there is currently a lack of drugs with clear efficacy and safety for controlling the progress of myopia, and there is an unmet clinical need in the field of this disease.

It is known that bendazac lysine (BDL) or bendazac has analgesic, antipruritic, anti-necrotic, cholagogic, and therapeutic effects on dyslipidemia. Bendazac lysine is clinically used to treat cataracts by preventing lens protein degeneration.

The present inventors have surprisingly found that bendazac or bendazac lysine have the effect of treating, preventing, or slowing the progress of myopia and myopia-related symptoms, which show that these compounds can effectively control, inhibit, delay, or slow down the progress of myopia and finally used to manufacture a preparation or a pharmaceutical composition for preventing and treating myopia, and such compound has the advantages of high drug safety and less adverse reactions.

In view of the above findings, the present application provides the use of bendazac lysine or bendazac, or an optical isomer thereof, a racemate thereof, a solvate thereof, a pharmaceutically acceptable salt thereof, a prodrug thereof, a metabolite thereof, an analog thereof or a derivative thereof, a crystalline compound thereof, or a combination of these substances, in one or at least two of the following:
(a) preventing and/or treating myopia and myopia-related symptoms;
(b) inhibiting, slowing elongation of the axis length of eye(s) and/or increase in the length (depth) of the vitreous chamber of eye(s) in an individual with myopia or a tendency to develop myopia;
(c) increasing choroidal thickness and/or inhibiting a decrease in choroidal thickness in an individual with myopia or a tendency to develop myopia;
(d) delaying, reducing, or treating an abnormal development of eyeball associated with ametropia;
(e) enabling an individual to obtain clearer distance vision without using or replacing lenses (such as myopia frame glasses or OK lenses) or relying on other vision correction means (such as refractive surgery);
(f) controlling, inhibiting, delaying, or slowing down the progress (speed) of diopter (continuously) becoming negative in an individual with myopia or a tendency to develop myopia;
(g) preventing and/or treating myopia and related symptoms thereof in combination with surgery (such as refractive correction surgery, myopia corneal laser surgery, lens surgery) or other vision correction means (such as corneal contact lenses);
(h) preventing and/or treating myopia and myopia-related symptoms in combination with one or more other drugs;
(i) reducing a distance between a retina and a lens, preferably reducing the distance between the retina and the lens in an individual with myopia or a tendency to develop myopia;
(j) reducing a degree of myopia, or treating myopia, or treating nearsightedness, or controlling a progression of myopia, or correcting myopia, or alleviating myopia, or preventing myopia in adolescents;
(k) inhibiting or treating myopia caused by lens lesions;
(l) maintaining dioptric stability in an individual whose eyeball is at a developmental stage, in particular controlling the elongation speed of the axial length in order to maintain a match between it and the refractive component(s), said match preferably being able to maintain emmetropia or maintain emmetropia as far as possible;
(m) maintaining dioptric stability in an individual whose eyeball is at a developmental stage, in particular controlling the elongation speed of the axial length in order to maintain a match between it and the refractive component(s), said match preferably may avoid the occurrence of myopia or inhibit an increase in the degree of myopia;
(n) manufacturing a pharmaceutical composition, preparation, or device to fulfill at least one of the uses of (a) to (m) above.

In some embodiments, bendazac lysine or bendazac, or the optical isomer thereof, the racemate thereof, the solvate thereof, the pharmaceutically acceptable salt thereof, the prodrug thereof, the metabolite thereof, the analog thereof or the derivative thereof, the crystalline compound thereof, or the combination of these substances acts as a sole active ingredient or as a main active ingredient.

In some embodiments, a content of the sole active ingredient or main active ingredient accounts for 50% or mroe, 60% or more, 70% or more, 80% or more, 90% or more or 100% of total active ingredients, in which the percentage is a mass ratio or a molar ratio.

In some embodiments, these substances or the combination thereof and the one or more other drugs are formulated or designed in a manner of continuous administration, simultaneous administration, sequential administration, alternating administration, interval administration, or separate administration.

In some embodiments, the abnormal development of eyeball associated with ametropia is mainly developed during the juvenile phase of an individual (such as 2-28 years of age in humans), and is mainly induced by environmental factors or mainly caused by human factors (such as long-term near-distance reading, frequent use of electronic screens, persistent nearsightedness and lack of chance for farsightedness, improper use of refractive corrective glasses, side effects of drugs, obesity, trauma, poor lighting in the learning environment, lack of outdoor exercises), while genetic factors are secondary factors, concomitant factors, synergistic factors, or the abnormal development is a refractive dysplasia completely independent of genetic factors, mainly characterized in that in a state of accommodative relaxation, a focus falls in front of the retina after parallel light rays are refracted through a refractive system of eyes.

In some embodiments, the analog or derivative of bendazac lysine or bendazac is one of the following compounds (a)-(c):
(a) 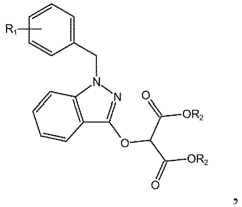 wherein R₁ is H, P (protium), D (deuterium), T (tritium), *p*-CH₃, *m*-F, *m*-Cl, or *p*-Cl; R₂ is H, P (protium), D (deuterium), T (tritium), K or Na;
(b) 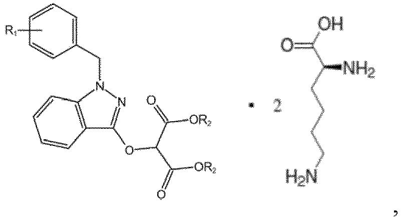 wherein R₁ is H, P (protium), D (deuterium), T (tritium), *p*-CH₃, *m*-F, *m*-Cl, or *p*-Cl; R₂ is H, P (protium), D (deuterium), T (tritium), K or Na;
(c) 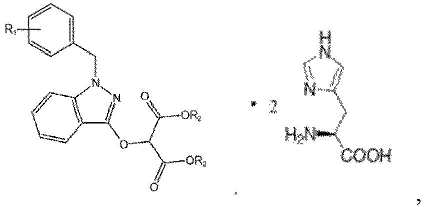 wherein R₁ is H, P (protium), D (deuterium), T (tritium), *p*-CH₃, *m*-F, *m*-Cl, or *p*-Cl; R₂ is H, P (protium), D (deuterium), T (tritium), K or Na.

In some embodiments, systemic administration (such as oral administration, intravenous drip), topical administration (such as eye drop, intravitreal injection, skin cream or ointment application, preferably, application with 3% skin ointment or ophthalmic ointment application), or parenteral administration (such as via mucosal administration, transdermal administration, microneedle administration), or non-invasive administration (such as by applying an ophthalmic ointment to the cornea or squeezing it into a sac formed by stretching of the lower eyelid), or non-invasive administration (such as using an ophthalmic spray) is used.

In some embodiments, the administrations (such as eye drop, oral administration) are used simultaneously, in combination, alternately, at intervals, separately, or one of them selected for use.

In some embodiments, wherein a preparation for topical administration includes but not limited to aqueous, oily or suspension preparation, that can incorporate pharmacologically and/or physiologically active ingredients such as a mydriatic component, decongestant component, eye muscle (such as ciliary muscle) regulating component, anti-inflammatory component, astringent component, antihistamine component, antiallergic component, hepatoprotective (avoiding or attenuating hepatotoxicity) component, blood-retinal barrier enhancing component (making it more difficult for compounds to penetrate through the physiological barrier), local anesthetic component, glaucoma treatment component, cataract treatment component, and the like.

In some embodiments, a concentration or a ratio of these substances or the combination thereof in the pharmaceutical composition, preparation, or device is at least not less than 0.01%, preferably 0.01% to 0.8%, preferably 0.05% to 0.5%, more preferably 0.1%; or the concentration or the ratio of these substances or the combination thereof is less than 0.01%, in which the percentage being expressed as mass/volume concentration (ratio) or mass ratio or molar (number) ratio, preferably the concentration is a concentration for use or storage.

In some embodiments, the concentration of these substances or combinations thereof is, for example, 0.01% to 0.05%, 0.05% to 0.1%, 0.1% to 0.5%, in which the percentages being expressed as mass/volume concentration (ratio), preferably the concentration being a concentration for use or storage.

In some embodiments, the pharmaceutical composition or preparation can be an injection, tablet, lyophilized powder injection, capsule, effervescent tablet, chewable tablet, buccal tablet (lozenge), granule, ointment, syrup, oral liquid, aerosol, nasal drops, external preparation, oral preparation and the like; preferably ophthalmic dosage form, including but not limited to eyedrops (eye drops), eye ointment, eye spray, implant, ophthalmic gel, ophthalmic patch, ophthalmic microsphere, ophthalmic sustained-release preparation, periocular injection or intraocular injection; or a free solution, oil-water mixture, suspension, liniment, lotion, cream, drop, electuary, spray, ointment, patch, paste, pill, suppository or emulsion..

In some embodiments, the individual with myopia or a tendency to develop myopia is a human being, and can be a child, adolescent, middle-aged person or elderly person, preferably a person aged 3 to 26, more preferably a person aged 6 to 18; or an adult or minor, preferably those whose eyes (eyeballs) are still in the growth and development stage; or a school-age people, preferably students in grades 1 to 12.

In some embodiments, the myopia is refractive myopia or axial myopia; congenital myopia (birth or preschool myopia), early-onset myopia (under 14 years old), delayed myopia (16 to 18 years old), late-onset myopia (after adulthood); low myopia (mild myopia), moderate myopia, high myopia (severe myopia); pseudo-myopia, true-myopia, semi-true and semi-pseudo (mixed) myopia; myopia in children and/or adolescents (preferably the age of the population is 3 to 26, more preferably the age of the population is 6 to 18), myopia in minors, myopia in adolescents, myopia in adults, myopia in the elderly; simple myopia, pathological myopia; axial simple myopia, simple axial myopia; axial myopia in children and/or adolescents (preferably the age of the population is 3 to 26, more preferably the age of the population is 6 to 18); axial myopia in school age and pre-school population; primary myopia, secondary myopia; primary myopia in children and/or adolescents (preferably the age of the population is 3 to 26, more preferably the age of the population is 6 to 18); progressive myopia in children and/or adolescents (preferably the age of the population is 3 to 26, more preferably the age of the population is 6 to 18); curvature myopia, index myopia, positional myopia, bending myopia; myopia caused by long-term short distance use of eye(s), myopia and pseudo-myopia caused by asthenopia, negative diopter caused by adverse drug reactions, nearsightedness, myopia caused by reading, myopia caused by the use of electronic products such as mobile phones, myopia caused by mismatch of refractive media (components), refractive myopia, myopia caused by abnormal refractive development, myopia caused by excessive eyeball growth, myopia caused by unhygienic use of eyes, myopia caused by imaging focus of distant object falling in front of the retina caused by various reasons, myopia caused by poor or ineffective treatment of atropine, myopia caused by insufficient outdoor exercises, and accommodative tension myopia, childhood myopia, or myopia dominated by environmental factors.

In some embodiments, the aforementioned myopia includes or does not include myopia or myopia symptoms caused by lens lesions.

In some embodiments, the myopia-related symptoms or signs include complications resulting from myopia, such as complications resulting from high myopia, muscae volitantes, glaucoma, posterior scleral staphyloma, retinal detachment, retinal tear, amblyopia, macular hemorrhage, choroidal neovascularization, choroidal atrophy, macular degeneration or maculopathy, visual field loss, progressive or sudden decrease in vision (especially near vision), ocular soreness and/or pain, night blindness, astigmatism, anisometropia, blindness, vitreous liquefaction, vitreous opacity, strabismus, frequent blinking, frequent eye rubbing, anisometropia, blurred vision when looking at distant objects, squinting or partially closing eyelids to see distant objects clearly, headache caused by asthenopia, difficulty in vision when driving, especially at night (night myopia), retinal atrophy and degeneration (bleeding and tears), subretinal neovascularization, and eyeball atrophy.

In some embodiments, the pharmaceutical composition, preparation, or device further includes a medicinal preparation or medicament including, but not limited to, a drug for treating myopia (e.g. atropine, dibazole, pirenzepine, muscarinic antagonist, 7-methylxanthine (7MX), aminobenzylamine, indoleamine, timolol maleate, epinephrine, pirenzepine, pyrazine, pybenpine, pibenpine, pyenzepine, methylamine, chlorisondamine, acetylcholinesterase inhibitor, dopamine agonist, gamma-aminobutyric acid, naloxone, glucagon, retinoic acid, etc.), M receptor blocker (such as a blocker or antagonist or inhibitor for the M3 receptor),, polyunsaturated fatty acid (such as DHA, EPA), salidroside, formononetin, prazosin, homatropine, anisodamine (racemic), tropicamide, nicotinic acid, piracetam, salvia miltiorrhiza extract, safflower extract, fish oil, bear bile extract, vitamin, ATP, non-selective adenylate antagonist, vasodilator, mydriatic, smooth muscle relaxant, vasospasm preventing drug, collagen metabolism regulating drug, anti-allergic drug, anti-inflammatory drug, liver-protecting drug, therapeutic component for ophthalmic disease, topical ophthalmic anesthetic, or ophthalmic preparation.

In some embodiments, the pharmaceutical composition, preparation, or device further includes a pharmaceutical preparation or medicament including bendazac or various salt forms thereof, or bendazac lysine or various salt forms thereof. This means that combinations of "bendazac or various salt forms thereof' with bendazac lysine, or an optical isomer thereof, a racemate thereof, a solvate thereof, a pharmaceutically acceptable salt thereof, a prodrug thereof, a metabolite thereof, an analog or derivative thereof, or a crystalline compound thereof are ultimately formed in these pharmaceutical compositions, preparations, or devices; or combinations of "bendazac lysine or its various salt forms" with bendazac, or an optical isomer thereof, a racemate thereof, or solvate thereof, a pharmaceutically acceptable salt thereof, a prodrug thereof, a metabolite thereof, an analog or derivative thereof, or a crystalline compound thereof are ultimately formed in these pharmaceutical compositions, preparations, or devices.

In some embodiments, a pharmaceutical preparation or drug and the pharmaceutical composition, preparation or device of the present application are administered contemporaneously, such as concurrently or sequentially administrated during a particular course of administration (treatment), administrated on the same day, administrated in the same week, administrated in the same month, administrated in the same year; or alternately administrated at intervals, such as alternately administrated at intervals of 4 hours, alternately administrated at intervals of 12 hours, alternately administrated every other day, alternately administrated every other week, alternately administrated every other month and alternately administrated every other year.

In some embodiments, the one or more other drugs include, but are not limited to, a drug for treating myopia (e.g. atropine, dibazole, pirenzepine, muscarinic antagonist, 7-methylxanthine (7MX), pirenzepine, aminobenzylamine, indoleamine, timolol maleate, epinephrine, pyrazine, pybenpine, pibenpine pyenzepine, methylamine, chlorisondamine, acetylcholinesterase inhibitor, dopamine agonist, gamma-aminobutyric acid, naloxone, glucagon, retinoic acid, etc.), M receptor blocker (such as a blocker or antagonist or inhibitor for the M3 receptor), polyunsaturated fatty acid (such as DHA, EPA), salidroside, formononetin, prazosin, homatropine, anisodamine (racemic), tropicamide, nicotinic acid, piracetam, salvia miltiorrhiza extract, safflower extract, fish oil, bear bile extract, vitamin, ATP, non-selective adenylate antagonist, vasodilator, smooth muscle relaxant, vasospasm preventing drug, collagen metabolism regulating drug, anti-allergic drug, anti-inflammatory drug, liver-protecting drug, therapeutic component for ophthalmic disease, topical ophthalmic anesthetic, mydriatic, or ophthalmic preparation or drug.

In some embodiments, one or more other drugs may also include bendazac or various salt forms thereof, or bendazac lysine or various salt forms thereof. This means that combination administration of these substances of the present application and these drugs may ultimately take the form of "bendazac or its various salt forms" in combination with bendazac lysine, an optical isomer thereof, a racemate thereof, a solvate thereof, a pharmaceutically acceptable salt thereof, a prodrug thereof, a metabolite thereof, an analog thereof or a derivative thereof, a crystalline compound thereof, or combination administration of these substances of the present application and these drugs may ultimately take the form of "bendazac lysine or various salt forms thereof' in combination with bendazac, or an optical isomer thereof, a racemate thereof, a solvate thereof, a pharmaceutically acceptable salt thereof, a prodrug thereof, a metabolite thereof, an analog thereof or a derivative thereof, or a crystalline compound thereof.

In some embodiments, the combination -administration means that compared to these substances of the present application, the one or more other drugs is administered contemporaneously, such as concurrently or sequentially administrated during a particular course of administration (treatment), administrated on the same day, administrated in the same week, administrated in the same month, administrated in the same year; or alternately administrated at intervals, such as alternately administrated at intervals of 4 hours, alternately administrated at intervals of 12 hours, alternately administrated every other day, alternately administrated every other week, alternately administrated every other month and alternately administrated every other year.

In some embodiments, the preparation can also be an oral product such as a health product, food product, dietary supplement, nutritional product, drink, or a cosmetic product; wherein the cosmetic product can be one or a combination of a free solution, oil-water mixture, suspension, liniment, lotion, spray, cream, drop, electuary, ointment, paste, pill, suppository, emulsion, and patch.

In some embodiments, the device is an instrument, apparatus, consumable, system, medical device, health care product or product for changing the appearance of the eye, capable of releasing a drug or having a drug delivery function or a potential drug delivery capability, such as corneal contact lens(es), glasses, intraocular lens(es), suture, OK lens(es) cleaning (maintenance) system, eye patch, eyesight improving patch, cosmetic lens(es), microneedle, eye spray system, eye massager (myopia massager), eye fumigator, ocular surface drug delivery device, intraocular drug delivery device, fundus drug delivery device, implanted pump, wearable equipment, acupoint massage instrument, eye relaxation equipment, myopia treatment instrument, or drug-device combination for myopia prevention and control. In some embodiments, the device may be referred to as an ophthalmic device.

### Terms and definitions

Unless defined otherwise, all technical and scientific terms used herein have the same meaning as commonly understood by one of ordinary skill in the art to which this application belongs. The terms used herein are for the purpose of describing particular embodiments only and are not intended to be limiting to this application. The terms "comprises" and "comprising" and any variation thereof in the description and claims of the present application and the preceding description of the figures are intended to cover a non-exclusive inclusion.

Reference herein to "one or more" means that at least one of the elements is present. A plurality of such elements may be present unless specifically limited otherwise.

In the description of this application, the term "and/or", describing an associated relationship of associated objects only, which means that three kinds of relationships may exist, for example, A and/or B, may mean three conditions: A is present alone, A and B are present together, and B is present alone. In addition, the character "/", as used herein, generally indicates that the associated object is an "or" relationship.

Reference herein to "example" or "embodiment" means that a particular feature, structure, or characteristic described in connection with the example (or embodiment) may be included in at least one example (or embodiment) of the present application. The appearances of the phrase in various places in the specification are not necessarily all referring to the same example (or embodiment), nor are separate or alternative examples (or embodiment) mutually exclusive of other examples (or embodiments). It is to be explicitly and implicitly understood by one of ordinary skill in the art that the example (or embodiment) described herein may be combined with other examples (or embodiments).

In this specification and in the appended claims, unless the context clearly dictates otherwise, the singular forms, including the singular forms "a", "an", and "the/said" are also specifically covers the plural referents of the terms to which they refer. Additionally, as used herein, the word "alternatively/or" refers to the "inclusive" meaning of "and/or" and rather than the "exclusive" meaning, unless specifically stated otherwise.

As used herein, a reference to a numerical range of a variable is intended to convey that the application can be practiced with the variable equal to any value within the range. Thus, for a variable that is inherently discontinuous, the variable can be equal to any integer value within the numerical range, including the endpoints of the range. Similarly, for a variable that is inherently continuous, the variable can be equal to any real value within the numerical range, including the endpoints of the range. For example, a variable described as having a value between 0 and 2 can be 0, 1, or 2 for inherently discontinuous variables and can be 0.0, 0.1, 0.01, 0.001, or any other real value for inherently continuous variables.

As used herein, "about" will be understood by one of ordinary skill in the art and will vary to some extent depending on the context in which it is used. If the use of the term is not clear to one of ordinary skill in the art, in the context in which the term is used, "about" will indicate a numerical value within the range of plus or minus 10% of the recited value.

The terms "individual" and "subject" include humans as well as non-human animals, including, for example, farm animals such as sheep, pigs, cattle, and horses; pet animals such as dogs and cats; laboratory animals such as mice, rats, and non-human primates. In a preferred embodiment, the mammal is a human.

As used herein, "administer or administering or administration of' a compound, preparation, test article, or drug to a subject includes any way of introducing or administering the compound to a subject to perform its intended function. "Administration" can be performed by any suitable route, including but not limited to oral, intraocular, intranasal, parenteral (by intravenous, intramuscular, intraperitoneal, or subcutaneous) or topical administration. "Administration" includes self-administration and administration by others.

In the administration mode herein, "used separately" means that only one mode of administration is used at each stage of the overall course of administration and the mode of administration can be changed (but not alternated) in different stages of the course of administration.

In the administration mode herein, the term "one of them selected for use" means that only one route of administration is used and is not altered in the overall course of administration.

As used herein, the term "amino acid" includes naturally occurring amino acids and synthetic amino acids as well as amino acid analogs and amino acid mimetics that act in a manner similar to a naturally occurring amino acid. The naturally occurring amino acids are those encoded by genetic codes and those subsequently modified, such as hydroxyproline, γ-carboxyglutamic acid, and o-phosphoserine. Amino acid analogs refer to compounds having the same basic chemical structure as a naturally occurring amino acid, i.e. an α-carbon, a carboxyl group, an amino group, and an R group bonded to hydrogen. The amino acid analogs are such as homoserine, norleucine, methionine sulfoxide, methionine methyl sulfonium. Such analogs have modified R-groups (such as norleucine) or modified peptide main chains, but maintain the same basic chemical structure as naturally occurring amino acids. Amino acid mimetics refers to chemical compounds that have structures that are different from the general chemical structure of amino acids, but that function in a manner similar to naturally occurring amino acids. Amino acids may be referred to herein by commonly known three-letter symbols or one-letter symbols recommended by the IUPAC-IUB Biochemical Nomenclature Committee.

As used herein, the term "effective amount" refers to an amount sufficient to achieve the desired therapeutic and/or prophylactic effect, e.g. to cause prevention or alleviation of a disorder associated with an ophthalmic disorder. The amount of composition administered to a subject will depend on the type and severity of the disease and the nature of the individual, such as general health, age, sex, weight, ethnicity, degree of myopia, rate of progression of myopia, and tolerance to drugs. The amount will also depend on the degree, severity and type of disease, as well as the treatment regimen prescribed by the practitioner, such as a physician. Professional technicians will be able to determine the appropriate dosage based on these and other factors. The pharmaceutical compositions can also be administered in combination with one or more other therapeutic compounds, biopharmaceuticals, and therapeutic molecules (e.g. polypeptides). In the methods described herein, the bendazac, bendazac lysine compounds, or pharmaceutical compositions containing the same can be administered to a subject having one or more symptoms or signs of an ophthalmic disorder. For example, a "therapeutically effective amount" of bendazac lysine refers to an average level that minimally alleviates the physiological effects of ophthalmic disorders, preferably an average level that minimally controls the physiological effects of the progression of myopia.

As used herein, the terms "preparation", "pharmaceutical composition", and "composition" are used interchangeably and may refer to a mixture of two or more compounds, elements, or molecules. In some aspects, the terms "preparation", "pharmaceutical composition", and "composition" may be used to refer to a mixture of one or more active agents (active ingredients) with a carrier or other excipient. The compositions can take almost any physical form, including solids, liquids (e.g. solutions), or gases.

The term "dosage form" may include one or more preparations or compositions provided in a form for administration to a subject. For example, an injectable dosage form can be a preparation or composition prepared in a manner suitable for administration by injection.

The term "pharmaceutically acceptable" as used herein means approved for use in animals, preferably humans, by regulatory authorities such as CFDA (China), EMEA (Europe) and/or FDA (US), and/or any other national administrative administration.

As used herein, an "ophthalmically acceptable carrier" is an ophthalmically acceptable solvent, suspension, or vehicle for the administration of a pharmaceutical composition to the eye of a subject. The carrier may be solid or liquid. The carrier is "ophthalmically acceptable" in the sense that the carrier is suitable for administration to the eye without causing any substantial adverse reaction.

As used herein, the term "simultaneously" means that at least two active ingredients are administered by the same or different routes (e.g. orally and eye drops) and at the same time or substantially the same time when administered therapeutically; or administering and performing surgery at or substantially at the same time; alternatively, or administration and application of the therapeutic devices at the same time or substantially the same time.

As used herein, the term "separately" means limited to one mode or substance at the same time or at substantially the same time when administered therapeutically, e.g. only one active ingredient is administered.

The term "sequentially" as used herein means that at least two active ingredients are administered at different times, by the same or different routes of administration when administered therapeutically. More specifically, "sequential application" refers to the one of the active ingredients is completely administered before the start of the administration of the other active ingredient. Thus, one active ingredient may be administered several seconds, minutes, hours, or days before the other active ingredient is administered.

As used herein, the terms "treating", "controlling", "inhibiting", "delaying", "reducing", "preventing and controlling", "preventing", or "slowing (down)" refer to both therapeutic treatment and prophylactic or preventative measures, wherein the object is to prevent or slow down (lessen) a targeted condition or disorder, or even eliminate or reverse it. For example, the subject exhibits observable and/or measured avoidance, diminishment, and disappearance of one or more symptoms and signs of the ophthalmologic disorder, or slowing of disease progression, after receiving a therapeutic amount of a bendazac lysine compound or a pharmaceutical composition comprising the same according to the methods described herein, an ophthalmologic disorder in a subject is successfully treated. It will also be understood that the various modes of treating or preventing a medical condition described herein are intended to mean "significant", which includes both complete treatment or prevention as well as less than complete treatment or prevention, wherein some biologically or medically relevant result is achieved. For example, in some embodiments "treating" does not require 100% elimination or prevention of myopia or myopia-related symptoms. In some embodiments, myopia or myopia-related symptoms that are "treated" according to the methods of the present disclosure are reduced, inhibited, discontinued, prevented, and/or reversed, e.g. by at least about 5%, by at least about 10%, or by at least about 20%, as compared to the levels observed in the absence of the compositions or methods of the present application (e.g. in a biologically-matched control subject, individual, or specimen that has not been exposed to a compound of the compositions or methods of the present application). In some embodiments, the myopia or myopia-related symptoms are treated by at least about 30%, at least about 40%, at least about 50% or at least about 60%, at least about 70%, at least about 80%, at least about 90% or more (about 100%) as compared to myopia or myopia-related symptoms in the absence of a compound of the methods of the present application.

As used herein, the term "(individual with) a tendency to develop myopia" may be a condition in which a decrease in diopter level has occurred but has not yet become negative; it may also be a susceptible or high-risk group for myopia predicted or considered by an authority or professional physician; it may also be individuals with family history of myopia; it may also include individuals who receive more near vision information but lack distance vision opportunities; it may also refer to the occurrence (disease) rate of myopia or the severity of myopia not less than the average level once myopia occurs; it may also refer to the situation where the adverse reaction after taking the drug or the postoperative risk of receiving the surgery includes the decrease of diopter; it may also mean that the individual will develop myopia or a diopter drop below zero if the individual is not intervened with a drug or other myopia treatment (prevention and control) means.

"Ophthalmic composition" or "ophthalmic preparation" or "ophthalmological preparation" means an ophthalmic composition, or an ophthalmic pharmaceutical composition, or an ophthalmic pharmaceutical product; or a pharmaceutical part of a drug, preparation, cosmetic, health-care product, drug-device combination, or device for preventing and/or treating of ocular diseases, protection of vision, maintaining, improving, avoiding, slowing or reversing vision impairment.

"Fish oil" refers to lipid materials derived from higher animals, especially fish (e.g. cod, salmon), squid, seal, and particularly polyunsaturated fatty acids therein, including but not limited to omega-3 unsaturated fatty acids, DHA, EPA, DPA, ALA, nisinic acid, stearidonic acid, eicosatetraenoic acid, or combinations thereof.

"Analog" refers to a structural derivative of a parent compound (e.g. bendazac or bendazac lysine as referred to herein) that differs in only one element (including isotopes) from the parent compound.

As used herein, the term "derivative" of a compound includes any molecule that is functionally and/or structurally related to the compound, such as an acid, amide, ester, ether, acetylated, hydroxylated, or alkylated (C₁-C₆) variant of the compound, halide, deuteride, and the like. The derivative should have a Tanimoto similarity index with the parent drug of greater than 0.4, preferably greater than 0.5, more preferably greater than 0.6, even more preferably greater than 0.7. The Tanimoto similarity index is widely used to measure the degree of structural similarity between two molecules. The Tanimoto similarity index can be calculated by software available online such as Small Molecule Subgraph Detector (http://www.ebi.ac.uk/thomton-srv/software/SMSD/). Preferred derivatives should be both structurally and functionally related to the parent compound, i.e. they should also retain at least part of the activity of the parent drug, e.g. a bendazac lysine derivative or analog as described in the reference Synthesis and biological evaluations of novel bendazac lysine analogs as potent anticataract agents (Bioorganic & Medicinal Chemistry Letters, 20, 2115-2118,2010), more preferably they should have a regulatory effect on refractive development. Furthermore, a "derivative" also includes a metabolite of a drug, e.g. a molecule that, after administration to an organism, is typically produced by (biochemical) modification or processing of the drug by a specialized catalytic system, and which exhibits or retains the biological activity of the drug. Metabolites have been disclosed to be responsible for most of the therapeutic effects of the parent drugs.

As used herein, a "metabolite" refers to a modified or processed drug that retains at least part of the activity of the parent drug, preferably has an inhibitory effect on Aldose Reductase (AR) activity or has an effect on the treatment, prevention or slowing of progression of myopia and related symptoms.

The term "therapeutically acceptable salt", as used herein, represents salts or zwitterionic forms of the compounds disclosed herein that are water or oil-soluble or dispersible and therapeutically acceptable, as defined herein. The salts can be prepared during the final isolation and purification of the compounds or separately by reacting the compound in the appropriate free base form with a suitable acid. Representative acid addition salts include acetate, adipate, alginate, L-ascorbate, aspartate, benzoate, benzenesulfonate (besylate), bisulfate, butyrate, camphorate, camphor sulfonate, citrate, digluconate, formate, fumarate, gentisate, glutarate, glycerol phosphate, glycolate, hemisulfate, heptanoate, hexanoate, hippurate, hydrochloride, hydrobromate, hydroiodide, 2-hydroxyethanesulfonate (isethionate), lactate, maleate, malonate, DL-mandelate, mesitylene sulfonate, methanesulfonate, naphthalene sulfonate, niacinate, 2-naphthalene sulfonate, oxalate, pamoate, pectinate, persulfate, 3-phenylpropionate, phosphonate, picrate, pivalate, propionate, pyroglutarate, succinate, sulfonate, tartrate, L-tartrate, trichloroacetate, trifluoroacetate, phosphate, glutamate, bicarbonate, para-toluenesulfonate (p-tosylate), and undecanoate. In addition, basic groups in the compounds disclosed herein can be quaternized with the following:: methyl, ethyl, propyl, and butyl chlorides, bromides and iodides; dimethyl, diethyl, dibutyl, and diamyl sulfate; decyl, lauryl, myristyl and steryl chlorides, bromides and iodides; and benzyl and phenethyl bromides. Examples of acids that can be employed to form therapeutically acceptable addition salts include inorganic acids such as hydrochloric, hydrobromic, sulfuric, and phosphoric, and organic acids such as oxalic, maleic, succinic, and citric. Salts may be formed by the coordination of the compounds with alkali or alkaline earth metal ions. Thus, the present application includes sodium, potassium, magnesium, and calcium salts and the like of the compounds disclosed herein.

The term "these substances" in this application means bendazac lysine or bendazac, or an optical isomer thereof, or a racemate thereof, or a solvate thereof, or a pharmaceutically acceptable salt thereof, or a prodrug thereof, or a metabolite thereof, or an analog or derivative thereof, a crystalline compound thereof.

"Myopia" means that in a state of accommodative relaxation, parallel light rays are collected in front of the retina after passing through the refractive system of the eye. According to the causes of refraction, it is divided into refractive myopia and axial myopia. Their clinical manifestations are blurred distant vision and good near vision. Long-distance vision often fluctuates in the early stage of myopia, because no or less adjustment is needed in near vision, so the convergence function is correspondingly weakened, and it is easy to cause exophoria or exotropia. Myopia-related symptoms also include poor vision at night, mosquito, flashing sensation, etc. and can occur with varying degrees of fundus changes, myopia arc plate and macular hemorrhage, or subretinal neovascularization, irregular white atrophic spots, or pigmented round black spots (Fuchs spots), lattice degeneration and cystic degeneration around the retina, vitreous liquefaction, opacity, posterior vitreous detachment, etc. at a younger ager. The risk of retinal hole and detachment is higher than normal people, scleral staphyloma is often formed due to the long anteroposterior diameter of the eyeball, prominent eyeball, and expanded posterior pole of the eyeball. Patients with the above clinical symptoms are referred to as pathological myopia.

Myopia can be a a disease that seriously weakens the eyes. A potential drawback (risk) of myopia is that the eyeball elongates slightly, causing the ocular lens to focus light from distant objects slightly in front of the retina. Thus, myopia is commonly referred to as shorten vision or near vision. In severe cases, this elongation of the eyeball can stretch and thin certain internal portions of the eye, which can increase the risk of retinal detachment, cataracts, glaucoma, blindness, etc. Thus, myopia is much more severe than simple shortening of vision.

Myopia involves axial elongation of the eye, affecting most of the population. Myopia usually develops during primary school age and progresses until the growth of the eye is complete. Despite the availability of corrective lenses, the development of myopia can result in increased visual deficits. The present disclosure recognizes the importance of pharmaceutical compositions and therapies for treating, preventing, preventing and controlling, controlling, inhibiting, mitigating, slowing (down), delaying, reducing, retarding and/or alleviating the onset and progression of myopia, particularly through pharmaceutical compositions, devices containing or delivering the pharmaceutical compositions and methods of their use, by facilitating administration or implementation, reducing potential side effects and providing therapeutic benefits or combinations thereof.

Many causes of myopia have been discussed and studied in the academic community, such as genetic susceptibility, prolonged book work or screen time, inadequate exposure to bright light, etc. Regardless of the underlying cause of myopia in a given situation, which may be one or more of the causes listed above, the elongated eyeball associated with myopia is debilitated for all people affected by such symptoms. Because the eyes grow in childhood and school age, myopia often occurs in school-age children and adolescents and can persist with these individuals throughout their lives. Thus, active medication interventions for individuals, such as school-age children and adolescents, can improve the quality of life of these individuals in their youth and for the rest of their lives.

The "an individual with myopia" or "an individual with a tendency to develop myopia" is a child, adolescent, middle-aged person, or elderly person, preferably population from 3 to 26 years old, more preferably population from 6 to 18 years old; or or a minor population, preferably those whose eyes (eyeballs) are still growing or developing; or a school-age group, preferably student population in grades 1 to 12 .

As used herein, the terms "myopia" and "nearsightedness" are used interchangeably. The meaning of "myopia" or "nearsightedness" in this application should be properly understood by the researchers in the field in the context in which it is used.

Specific types of "myopia" are refractive myopia or axial myopia; congenital myopia (birth or preschool myopia), early-onset myopia (under 14 years of age), delayed myopia (16-18 years of age), late-onset myopia (after adulthood); low myopia (mild myopia), moderate myopia, high myopia (severe myopia); pseudo-myopia, true-myopia; myopia in children and/or adolescents (preferably the age of the population is 3 to 26, more preferably the age of the population is 6 to 18), myopia in minors, myopia in adults, myopia in the elderly; simple myopia, pathological myopia; axial simple myopia, simple axial myopia; axial myopia in children and/or adolescents (preferably the the age of the population is 3 to 26, more preferably the age of the population is 3 to 26); axial myopia in school age and pre-school population; primary myopia, secondary myopia; primary myopia in children and/or adolescents (preferably the age of the population is 3 to 26,, more preferably the age of the population is 6 to 18); or progressive myopia in children and/or adolescents (preferably the age of the population is 3 to 26, more preferably the age of the population is 6 to 18); myopia caused by long-term short-distance use of eye(s), myopia and pseudo-myopia caused by asthenopia, negative diopter caused by adverse drug reactions, and nearsightedness.

"Axial myopia" refers to myopia in which the anterior-posterior diameter of the eyeball is too long (the axial length of the eye does not match other refractive components due to exceeding the normal range), while the refractive power (the refractive performance of other refractive components of the eye, such as cornea and lens) is substantially within the normal range.

"Refractive myopia" refers to myopia in which the axial length is substantially within the normal range, but is mainly caused by changes in the refractive properties of the refractive component(s).

"Pathological myopia", also known as degenerative myopia, is a degenerative disease in the fundus of the eye. Patients with myopia usually have high refractive power (generally greater than 600 degrees), significantly impaired visual function, and worse distance vision. In addition, visual field, light sensation, and contrast sensation are often abnormal, often accompanied by poor night vision (night blindness), muscae volitantes, floating objects, flashing sensation, etc. Such myopia is characterized by significant pathological changes, including retinal pigment epithelium thinning and atrophy, choroidal neovascularization and retinal detachment, macular degeneration, and severe blindness.

"Simple myopia" refers to myopia that occurs at school age and becomes stable with the cessation of development. The degree of myopia is below 600 degrees. There is no obvious pathological change in the fundus. It is also called acquired myopia. This type of myopia develops progressively, with appropriate lenses to correct vision to normal, and other visual function indicators are mostly normal.

"Primary myopia" specifically refers to myopia of unknown etiology and mechanism that cannot be determined by current diagnostic techniques. During its occurrence and development, non-transient functional-structural changes specific to myopia are manifested, including congenital myopia and acquired simple myopia.

"Concurrent/secondary myopia" refers to temporary myopia (such as toxic myopia, drug-induced myopia, traumatic myopia, diabetic myopia, and primary cataract myopia) etc. due to accommodation dysfunction or refractive index abnormality caused by internal and external factors. Such myopia is characterized by more definite inducing factors and repeated visual fluctuations. This type of myopia is often high in the elderly population.

"Axial simple myopia", sometimes also referred to as simple axial myopia, is simple myopia characterized by simple myopia in which the imaging focus is located in front of the retina due to the elongation of the axis length of the eye and/or increased depth of the vitreous chamber. This type of myopia is characterized by substantially normal refractive tissue (e.g. corneal curvature), which is the most common type of myopia in children and adolescents and occurs mostly in the population from 2 to 30 years old.

"Progressive myopia" refers to a type of myopia in which the diopter continues to decrease over time or with the age of the individual, and which, without intervention, will generally eventually develop to high myopia.

"Moderate myopia" generally refers to myopia above 300 degrees and below 600 degrees.

"Curvature myopia" is myopia caused solely by an increase in the curvature of the cornea or lens.

"Index myopia", which is a myopia mainly caused by an increase in refractive power caused by an increase in aqueous humor and lens refractive index, which belongs to refractive myopia.

"Accommodative tension myopia" is a myopia caused by regulating tension or regulating spasms due to excessive visual proximity load of the eyeball and excessive regulation of ciliary muscle.

"Myopia caused by lens lesions" refers to a type of myopia in which changes in the structural parameters or internal structure of the lens due to lens protein degeneration, accompanied by changes in some physical properties, such as thickness, hardness, and refractive index, which in turn result in parallel light rays are collected in front of the retina after passing through the diseased lens.

"Far vision or distane vision" is also referred to as naked far vision or naked distance vision. In medical practice, it refers to the vision measured when staring straight ahead with normal eyes open at the horizontal distance of 5 meters from the visual acuity chart, without wearing glasses and any auxiliary equipment (such as frame glasses, contact lenses, mydrioscope, and small hole lens) which has the effect of increasing vision.

The "myopia-related symptoms" comprise complications resulting from myopia, such as complications resulting from high myopia, muscae volitantes, glaucoma, posterior scleral staphyloma, retinal detachment, retinal tear, amblyopia, macular hemorrhage, choroidal neovascularization, choroidal atrophy, macular degeneration or maculopathy, visual field loss, progressive or sudden decrease in vision (especially near vision), ocular soreness and/or pain, night blindness, astigmatism, anisometropia, blindness, vitreous liquefaction, vitreous opacity, strabismus, frequent blinking, frequent eye rubbing, anisometropia, blurred vision when looking at distant objects, squinting or partially closing the eyelids to see distant objects clearly, headache caused by asthenopia, difficulty in vision when driving, especially at night (night myopia), retinal atrophy and degeneration (bleeding and tears), subretinal neovascularization, and eyeball atrophy

"Abnormal development of the eyeball" is a developmental abnormality of the size of the eyeball in children and adolescents (such as 3-26 years old), which is mainly characterized by excessive axial length of the eye, resulting in imaging focus of the parallel light rays is located in front of the retina after passing through the normal refractive system of the eye; or the abnormal development is mainly induced by environmental factors or mainly caused by human factors (such as long-term near-distance reading, frequent use of electronic screens, persistent nearsightedness, lack of chance for farsightedness, improper use of refractive corrective glasses), while the genetic factors are secondary factors, concomitant factors, synergistic factors, or the abnormal development is completely independent of genetic factors.

"Bendazac lysine" has a chemical name: L-lysine (1-benzyl-1H-indazol-3-yloxy) acetate, molecular formula: C₆H₁₄N₂O₂•C₁₆H₁₄N₂O₃, molecular weight: 428.49, and a structure formula as follows:

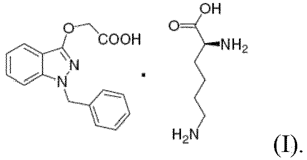

"Bendazac" has a structure formula as follows:

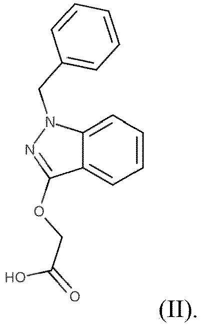

"Derivatives of bendazac or bendazac lysine" include optical isomers or racemates thereof or metabolites thereof (such as 5-hydroxybendazac) and the like, and specifically may also include but is not limited to those listed by Hong Shen, etc. (Bioorganic & Medicinal Chemistry Letters, 20, 2115-2118, 2010). Commercially available eye drops containing bendazac lysine (e.g. national medicine permission number H20063847) can be used, or bendazac lysine and derivatives thereof can be prepared using processes well known to those skilled in the art. An exemplary preparation method includes the following steps: a first step of synthesis is subjecting phenylhydrazine as the starting material to a benzylation reaction with benzyl chloride to obtain α-benzyl phenyl hydrazine; a second step of synthesis is subjecting α-benzyl phenylhydrazine to cyclization with urea at high temperature to obtain 3-hydroxy-1-benzylindazole; a third step of synthesis is subjecting 3-hydroxy-1-benzylindazole to carboxymethylation with chloroacetic acid to obtain bendazac, i.e. α-[(1-benzyl-1H-indazol-3-yl)oxy] acetic acid; and a fourth step of synthesis is subjecting bendazac to a salt formation with L-lysine in tetrahydrofuran followed by recrystallization in ethanol to obtain the final product bendazac lysine.

"Analogs or derivatives of bendazac lysine or bendazac", include e.g. (a)-(c):
(a) 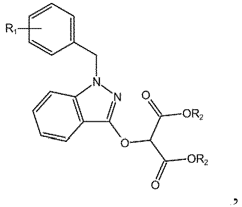 wherein R₁ is H, P (protium), D (deuterium), T (tritium), *p*-CH₃, m-F, *m*-Cl, or *p*-Cl; R₂ is H, P (protium), D (deuterium), T (tritium), K or Na;
(b) 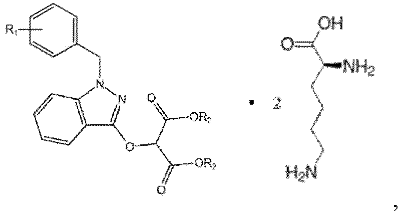 wherein R₁ is H, P (protium), D (deuterium), T (tritium), *p*-CH₃, *m*-F, *m*-Cl, or *p*-Cl; R₂ is H, P (protium), D (deuterium), T (tritium), K or Na;
(c) 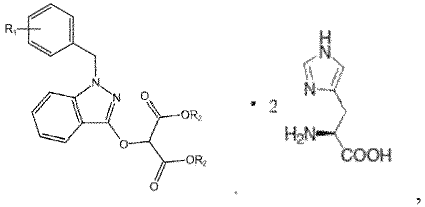 wherein R₁ is H, P (protium), D (deuterium), T (tritium), *p*-CH₃, *m*-F, *m*-Cl, or *p*-Cl; R₂ is H, P (protium), D (deuterium), T (tritium), K or Na.

The "preparation" can also be an oral product such as a health product, food product, dietary supplement, nutritional product, drink, or a cosmetic product; wherein the cosmetic product can be one or a combination of a free solution, oil-water mixture, suspension, liniment, lotion, spray, cream, drop, electuary, ointment, paste, pill, suppository, emulsion, and patch.

A "device" is an instrument, apparatus, consumable, system, medical device, health care product, or product for changing the appearance of the eye, capable of releaseing a drug or having a drug delivery function or a potential drug delivery capability, such as corneal contact lens(es), glasses, intraocular lens(es), suture, OK lens(es) cleaning (maintenance) system, eye patch, eyesight improving patch, cosmetic lens, microneedle, eye spray system, eye massager (myopic massager), eye fumigator, ocular surface drug delivery device, intraocular drug delivery device, fundus drug delivery device, implanted pump, wearable equipment, or drug-device combination for myopia prevention and control.

to the term "sole active ingredient or main active ingredient" in this application means that other active substances for the treatment of myopia is not contained or contained in a minor amount, in addition to bendazac lysine or bendazac, or an optical isomer thereof, a racemate thereof, a solvate thereof, a pharmaceutically acceptable salt thereof, a prodrug thereof, a metabolite thereof, an analog or derivatives thereof, a crystalline compound thereof, or a combination of these substances is present. For example, bendazac lysine or bendazac, or an optical isomer thereof, or a racemate thereof, a solvate thereof, a pharmaceutically acceptable salt thereof, a prodrug thereof, a metabolite thereof, an analog or derivative thereof, a crystalline compound thereof, or a combination of these substances accounts for 50% or more, 60% or more, 70% or more, 80% or more, 90% or more, or 100% of the total active ingredient, in which the percentage is a mass ratio or molar ratio; or bendazac lysine or bendazac, or an optical isomer thereof, a racemate thereof, a solvate thereof, a pharmaceutically acceptable salt thereof, a prodrug thereof, a metabolite thereof, an analog or derivatives thereof, a crystalline compound thereof, or a combination of these substances contributes 50% or more, 60% or more, 70% or more, 80% or more, 90% or more, or 100% of the therapeutic effect on myopia during administration.

Pharmaceutical composition therapy is a widely used and powerful strategy in medicine with the aim of achieving synergistic therapeutic effects, reducing dosage and toxicity, and minimizing or delaying the induction of resistance (Chou TC., "Drug combination studies and their synergy quantification using the Chou-Talalay method," Cancer Res. (2010)70: 440-6). The present disclosure identifies compounds, such as bendazac lysine, for use in the treatment, prevention, or amelioration of myopia and its related symptoms to improve the effect of reducing myopia (degree) or slowing myopia (progression) while avoide or minimize adverse side effects, such as those observed with atropine therapy. The pharmaceutical composition of the present application has a technical effect significantly superior to atropine in the treatment, prevention, or alleviation of myopia and related symptoms, and in addition to the improvement of diopter index, no adverse reactions such as photophobia and pupil dilation were observed in the experiment. In the experiment, no discomfort or ocular abnormality was observed in all animals that were administered, and further based on the existing clinical application basis of bendazac lysine or bendazac, it can be considered that it will have good drug safety in the clinical treatment of preventing or improving myopia and its related symptoms.

Through experiments, we have surprisingly found that bendazac and bendazac lysine significantly can slow down progression of diopter becoming negative in a form-deprivation guinea pig and a negative lens-induced guinea pig model of myopia, and significantly inhibit elongation of axial length. Based on this, it can be confirmed that bendazac and compounds in salt forms thereof have an effect of treating, preventing or controling of the progression of myopia in an animals, in particular humans, such as school-age children, adolescents or young adults.

The application provides a method for treating or preventing myopia and myopia-related symptoms in a subject, comprising administering to the subject a therapeutically effective amount of bendazac, bendazac lysine, and/or their therapeutically acceptable salts and derivatives thereof. Preferably, the bendazac or bendazac lysine is administered separately; preferably the bendazac and/or bendazac lysine is administered simultaneously or sequentially with the other drugs; preferably the bendazac and/or bendazac lysine is administered in the form of a pharmaceutical composition; preferably the pharmaceutical composition is prepared as an ophthalmic preparation; preferably the ophthalmic preparation further comprises a pharmaceutically acceptable carrier; preferably the carrier is an ophthalmically acceptable carrier.

The present application also provides a technique and method for inhibiting progression of axial myopia by inhibiting elongation of axial length of eye(s), including administering to a subject a therapeutically effective amount of bendazac, bendazac lysine and/or their therapeutically acceptable salts and derivatives thereof. Preferably, the bendazac or bendazac lysine is administered separately; preferably the bendazac and/or bendazac lysine is administered simultaneously or sequentially with the other drugs; preferably the bendazac and/or bendazac lysine is administered in the form of a pharmaceutical composition; preferably the pharmaceutical composition is prepared as an ophthalmic preparation; preferably the ophthalmic preparation further comprises a pharmaceutically acceptable carrier; preferably the carrier is an ophthalmically acceptable carrier.

The present application also provides a technique and method for reducing degree of myopia, including administering to a subject a therapeutically effective amount of bendazac, bendazac lysine, and/or their therapeutically acceptable salts and derivatives thereof. Preferably, the bendazac or bendazac lysine is administered separately; preferably the bendazac and/or bendazac lysine is administered simultaneously or sequentially with the other drugs; preferably the bendazac and/or bendazac lysine is administered in the form of a pharmaceutical composition; preferably the pharmaceutical composition is prepared as an ophthalmic preparation; preferably the ophthalmic preparation further comprises a pharmaceutically acceptable carrier; preferably the carrier is an ophthalmically acceptable carrier.

The present application also provides a hyperopia-enhancing agent for an individual with myopia, which is capable of improving distance vision of the individual with myopia by reducing the distance between the ocular imaging focus of the distant object and the retina. The hyperopia-enhancing agent comprises bendazac, bendazac lysine, and/or their therapeutically acceptable salts and derivatives thereof. Preferably, the myopia in the individual is refractive myopia. Preferably, the myopia in the individual is axial myopia.

The present application also relates to pharmaceutical compositions or methods for treating, preventing, or controling myopia and related symptoms thereof in an individual, such as an toddlers, school-age children, adolescents, or young adults. In some embodiments, the subject of the technical solutions of the present application is an adolescent, with an age range of 6-28 years, preferably 6-18 years, most preferably 12-18 years. In some embodiments, the subject of the technical solutions of the present application is an adult. In some examples, treating, preventing, or controling myopia and related symptoms thereof can include administering to a subject in need thereof a therapeutically effective amount of a pharmaceutical composition or dosage form.

In one embodiment, the pharmaceutical composition includes a therapeutically effective amount of a bendazac compound or salts and derivatives thereof, such as protium, deuterium, and tritium substitutions.

In other embodiments, for any one or more of the pharmaceutical compositions, devices, or methods of treatment, the pharmaceutical composition includes at least one of bendazac or a therapeutically acceptable salt thereof (e.g. bendazac lysine) or a derivative thereof; or the pharmaceutical composition includes bendazac lysine or a therapeutically acceptable salt or derivative thereof; preferably includes both bendazac and bendazac lysine; preferably includes both bendazac lysine and bendazac calcium hydrogen phosphate; preferably includes bendazac calcium hydrogen phosphate, preferably, further includes a pharmaceutically acceptable carrier; preferably, wherein the device delivers the pharmaceutical composition in a sustained-release manner.

In certain embodiments of the pharmaceutical composition, device, or method of treatment disclosed herein, the subject (patient) is treated for a period of time between about 0.5 months and 20 years, such as a period of at least 6 months, at least 1 year, at least 2 years, at least 3 years, at least 5 years, at least 9 years, or at least 13 years.

In other embodiments, the pharmaceutical composition, device, or method of treatment according to any one of the above embodiments and any one or more of the other embodiments herein, the pharmaceutical composition is an aqueous composition; preferably the aqueous composition has an osmotic pressure similar to or consistent with tears; or the pharmaceutical composition is an ophthalmic composition (such as an ophthalmic topical composition) or an ophthalmic preparation; preferably the ophthalmic preparation is an ophthalmic aqueous preparation, an ophthalmic gel preparation, an ophthalmic emulsion, an ophthalmic liposome, an ophthalmic ointment (preferably the ophthalmic ointment is an ophthalmic ointment, preferably the ophthalmic ointment contains petrolatum or liquid paraffin); alternatively, the pharmaceutical composition is an eye drop preparation, an eye spray preparation, a topical preparation, a nanoparticle suspension, or nano-disc, a sustained-release preparation, or a subconjunctival depot, etc.

In certain embodiments, the pharmaceutical compositions as disclosed herein may be ophthalmic aqueous preparations, such as in the form of eye drops. For example, an ophthalmic aqueous preparation as described herein may be packaged in an eye drop bottle and administered as drops. In certain embodiments, an ophthalmic aqueous preparation may be administered as a single administration (i.e. a single dose), which may include one, two, three or more drops into the eye of a patient. In certain embodiments, one dose of an ophthalmic aqueous preparation described herein is one drop of the aqueous composition from the eye drop bottle.

In certain embodiments, the pharmaceutical compositions as disclosed herein may be ophthalmic gel preparations. For example, the ophthalmic gel preparations may be packaged in an eye drop bottle and administered as drops. In certain embodiments, the ophthalmic gel preparation may be administered as a single administration (i.e. a single dose), which may include one, two, three, or more drops instilled into the eye of the patient. In certain embodiments, one dose of an ophthalmic gel described herein is one drop of the gel composition from the eye drop bottle.

In certain embodiments, the pharmaceutical compositions as disclosed herein may be ophthalmic ointment preparations. For example, the ophthalmic ointment preparations may be packaged in a tube or other squeezable container having a dispensing spout through which the ointment strip will be delivered. In certain embodiments, an ophthalmic ointment preparation may be administered as a single administration (i.e. a single dose), which may include one or more strips into a patient's eye. In certain embodiments, one dose of ophthalmic ointment is one strip of ointment composition dispensed through the orifice of the dispensing spout

In other embodiments, the pharmaceutical composition is an ophthalmic pharmaceutical composition contained within a contact lens blister package.

In some embodiments, the pharmaceutical composition is administered through a non-invasive administration route.

In other embodiments, the pharmaceutical composition, device or method of treatment according to any one of the above embodiments and any one or more of the other embodiments herein, wherein the device is preferably an ophthalmic device, e.g. it can be understood to mean an object placed on or present in the eye. The device may provide optical correction. Devices include but are not limited to, cosmetic lenses, contact lenses, ocular inserts, cornealonlays, cornealinlays, nanowafers, liposomes, nanoparticles, punctalonlays, or hydrogel matrices with microfluid reservoirs.

In other embodiments, the pharmaceutical composition is a sustained-release preparation included in a device.

In other embodiments, the pharmaceutical composition is included within a device.

In other embodiments, the pharmaceutical composition is an ophthalmic composition, and the ophthalmic composition is included within a device.

In other embodiments, the device needs to contain a pharmaceutical composition or is capable of delivering the pharmaceutical composition to the corresponding target tissue for the treatment of myopia.

In other embodiments, the device delivers the pharmaceutical composition in a sustained-release manner.

In other embodiments, the pharmaceutical composition is formulated as an ophthalmic composition for the treatment of an ophthalmic disorder or condition.

In other embodiments, the pharmaceutical composition is formulated as an ophthalmic composition for the treatment of anterior myopia, myopia (eye) or progression of myopia.

In other embodiments, the pharmaceutical composition is formulated as an ophthalmic composition for the treatment of high myopia, moderate myopia, or low myopia.

In other embodiments, the pharmaceutical composition is prepared as an ophthalmic composition for the treatment of axial myopia or refractive myopia.

In other embodiments, the pharmaceutical composition is formulated as an ophthalmic composition for the treatment of an individual (patient) diagnosed with (or with a risk of developing or predisposed to developing myopia) anterior myopia.

In other embodiments, the pharmaceutical composition is substantially uniformly distributed throughout the device.

In other embodiments, the device is contained within a contact lens blister package.

In other embodiments, the pharmaceutical composition is immersed in a device within a contact lens blister package.

Any method known to one of skill in the art may be used to contact a cell, organ or tissue with a compound such as bendazac or bendazac lysine. Suitable methods include in vitro, indirect in vivo, or in vivo methods. In vivo methods generally comprise administering a bendazac or/and bendazac lysine compound of the present application or a pharmaceutical composition containing the same to a mammal, preferably to a human. When used in vivo for treatment, the bendazac or/and bendazac lysine compounds or pharmaceutical compositions containing them can be administered to a subject in an effective amount (i.e. an amount having the desired therapeutic effect). The dosage and dosing regimen will depend on the extent of the ophthalmic disorder in the subject, the subject, and the medical history of the subject.

The compounds disclosed herein may also exist as prodrugs, as described in Hydrolysis in Drug and Prodrug Metabolism: Chemistry, Biochemistry, and Enzymology (Testa, Bernard and Mayer, Joachim M.Wiley-VHCA, Zurich, Switzerland 2003). The prodrug of the compound described herein is a modified form of the compound that is easy to produce chemical changes under physiological conditions to obtain the compound. In addition, prodrugs can be converted to the compounds by chemical or biochemical methods in an ex vivo environment. For example, prodrugs can be gradually converted to compounds when placed in a transdermal patch reservoir with a suitable enzyme or chemical reagent. Prodrugs are usually useful because in some cases, they may be easier to administer than the compounds or the parent drugs. For example, they can be bioavailable by oral administration, while parent drugs cannot. The prodrug may also have a higher solubility in pharmaceutical compositions than the parent drug. Many prodrug derivatives are known in the art, such as prodrug derivatives that rely on hydrolytic cleavage or oxidative activation of the prodrug. A non-limiting example of prodrugs is a compound administered as esters ("prodrugs") but then metabolically hydrolyzed into carboxylic acids (active entities).

The compounds disclosed herein can exist as therapeutically acceptable salts, including acid addition salts. Suitable salts include those formed with organic and inorganic acids, such acid addition salts are generally pharmaceutically acceptable; base addition salts may also be formed and are pharmaceutically acceptable. For a more complete discussion of salt preparation and selection, please refer to Pharmaceutical Salts: Properties, Selection, and Use (Stahl, P. Heinrich.Wiley-VCHA, Zurich, Switzerland, 2002).

Basic addition salts can be prepared during the final isolation and purification of the compounds by reacting a carboxy group with a suitable base such as the hydroxide, carbonate, or bicarbonate of a metal cation, or with ammonia or an organic primary, secondary, or tertiary amine. The cations of therapeutically acceptable salts include lithium, sodium, potassium, calcium, magnesium, and aluminum as well as nontoxic quaternary amine cations such as ammonium, tetramethylammonium, tetraethylammonium, methylamine, dimethylamine, trimethylamine, triethylamine, diethylamine, ethylamine, tributylamine, pyridine, N,N-dimethylaniline, N-methylpiperidine, N-methylmorpholine, dicyclohexylamine, procaine, dibenzylamine, N,N-dibenzylphenethylamine, 1-diphenylhydroxymethylamine, and N,N'-dibenzylethylene diamine. Other representative organic amines suitable for the formation of base addition salts include ethylenediamine, ethanolamine, diethanolamine, piperidine, and piperazine.

While it is possible to administer the compounds of the present application in the form of crude chemicals, it is also possible to provide them as pharmaceutical preparations. Accordingly, provided herein are pharmaceutical preparations including one or more certain compounds disclosed herein or one or more pharmaceutically acceptable salts, esters, prodrugs, amides, or solvates thereof, together with one or more pharmaceutically acceptable carriers thereof and optionally one or more other therapeutic ingredients. The carrier must be 'acceptable' in the sense that it is compatible with other components of the preparation and harmless to its recipient. Appropriate preparation is dependent upon the chosen route of administration. Any of the known techniques, carriers, and excipients that are suitable and understood in the art may be used; see, e.g. Remington's Pharmaceutical Sciences. The pharmaceutical compositions disclosed herein may be produced in any manner known in the art, e.g. by means of conventional mixing, dissolving, granulating, sugar coating, fine grinding, emulsifying, encapsulating, embedding or pressing processes.

The preparation includes those preparations suitable for oral, parenteral (including subcutaneous, intradermal, intramuscular, intravenous, intraarticular, and intramedullary), intraperitoneal, transmucosal, transdermal, rectal, and topical (including dermal, buccal, sublingual, ocular, intranasal, and intraocular) administration, and the most suitable route may depend upon for example the condition and disorder of the recipient. The preparations may conveniently be provided in unit dosage forms and can be prepared by any well-known method in the pharmaceutical field. Typically, these methods include the step of combining the compound or pharmaceutically acceptable salt, ester, amide, prodrug, or solvate thereof ("active ingredient") of the present application with a carrier that constitutes one or more auxiliary ingredients. In general, the preparation is prepared by uniformly and intimately combining the active ingredient with liquid carriers or finely divided solid carriers or both, and then, if necessary, shaping the product into the desired preparation.

The preparation of the compounds disclosed herein suitable for oral administration may be provided as discrete units such as capsules, cachets or tablets, each containing a predetermined amount of the active ingredient; as a powders or granules; as a solution or suspension in an aqueous liquid or a non-aqueous liquid; or as an oil-in-water emulsion or a water-in-oil emulsion. The active ingredient may also be provided as a bolus, syrup, electuary, or paste.

Pharmaceutical preparations that can be used orally include tablets, push-fit capsules made of gelatin, as well as soft, sealed capsules made of gelatin and plasticizer, such as glycerol or sorbitol. A tablet may be made by compression or molding, optionally with one or more auxiliary ingredients. Compressed tablets may be prepared by compressing the active ingredient in a free-flowing form such as a powder or granules, optionally mixed with a binder, inert diluent or lubricant, surface active or dispersing agent in a suitable machine. Molded tablets may be made by molding a mixture of the powdered compound moistened with an inert liquid diluent in a suitable machine. The tablets may optionally be coated or scored and may be formulated so as to provide sustained or controlled release of the active ingredient therein. All preparations for oral administration should be in dosages suitable for such administration. The push-fit capsules can contain the active ingredients in admixture with a filler such as lactose, a binder such as starch, and/or a lubricant such as talc or magnesium stearate, and, optionally, a stabilizer. In soft capsules, the active compounds may be dissolved or suspended in suitable liquids, such as fatty oils, liquid paraffin, or liquid polyethylene glycols. In addition, the stabilizer may be added. Sugar-coated pellet cores are provided with suitable sugar coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or sugar coatings to identifiy or to characterize different combinations of active compound doses.

Examples of fillers or diluents for oral pharmaceutical preparations such as capsules and tablets include but are not limited to, lactose, mannitol, xylitol, dextrose, sucrose, sorbitol, compressible sugar, microcrystalline cellulose (MCC), powdered cellulose, corn starch, pregelatinized starch, dextrate, dextran, dextrin, dextrose, maltodextrin, calcium carbonate, dibasic calcium phosphate, tribasic calcium phosphate, calcium sulfate, magnesium carbonate, magnesium oxide, poloxamers such as polyethylene oxide, and hydroxypropylmethyl cellulose. The fillers may have complex solvent molecules, such as where the lactose used is lactose monohydrate.

Examples of disintegrants for oral pharmaceutical preparations such as capsules and tablets include but are not limited to, sodium starch glycolate, sodium carboxymethyl cellulose, calcium carboxymethyl cellulose, croscarmellose sodium, povidone, crospovidone (polyvinylpyrrolidone), methylcellulose, microcrystalline cellulose, powdered cellulose, low-substituted hydroxypropyl cellulose, starch, pregelatinized starch, and sodium alginate.

In addition, glidants and lubricants can be used in oral pharmaceutical preparations to ensure a uniform blending of excipients durinh mixing. Examples of lubricants include but are not limited to, calcium stearate, glyceryl monostearate, glyceryl palmitostearate, hydrogenated vegetable oil, light mineral oil, magnesium stearate, mineral oil, polyethylene glycol, sodium benzoate, sodium lauryl sulfate, sodium stearyl fumarate, stearic acid, talc, and zinc stearate. Examples of glidants include but are not limited to, silicon dioxide (SiO₂), talc corn starch, and poloxamers. Poloxamers (or available from BASF Corporation) are A-B-A block copolymers where the A segment is a hydrophilic polyethylene glycol homopolymer and the B segment is a hydrophobic polypropylene glycol homopolymer.

Examples of binders for tablet include, but are not limited to, acacia, alginic acid, carbomer, sodium carboxymethyl cellulose, dextrin, ethyl cellulose, gelatin, guar gum, hydrogenated vegetable oil, hydroxyethyl cellulose, hydroxypropyl cellulose, hydroxypropylmethyl cellulose, copolyvidone, methyl cellulose, liquid glucose, maltodextrin, polymethacrylates, povidone, pregelatinized starch, sodium alginate, starch, sucrose, tragacanth, and zein.

The compounds may be formulated for parenteral administration by injection, e.g. by bolus injection or continuous infusion. The preparations for injection may be presented in unit dosage form, e.g. in an ampoule or a multi-dose container added with a preservative. The compositions may take such forms as suspensions, solutions, or emulsions in oily or aqueous vehicles, and may contain formulatory agents such as suspending, stabilizing, and/or dispersing agents. The preparation may be presented in a unit-dose or multi-dose container, such as sealed ampoule and vial, and may be stored in powder form or in a freeze-dried (lyophilized) state requiring only the addition of the sterile liquid carrier, for example, physiological saline or sterile pyrogen-free water, immediately before use. Extemporaneous injection solutions and suspensions may be prepared from sterile powders, granules, and tablets of the type previously described. In a preferred embodiment, the pharmaceutical composition of the present application is in the form of an injection, especially a syringe. Preferably, the pharmaceutical composition is administered by intraocular injection, more preferably by intravitreal injection into the vitreous.

Preparations for parenteral administration include aqueous and non-aqueous (oily) sterile injection solutions of the active compound, which may contain anti-oxidants, buffers, bacteriostats, and solutes that render the preparation isotonic with the blood of the intended recipient; and aqueous and non-aqueous sterile suspensions which may include suspending agents and thickening agents. Suitable lipophilic solvents or vehicles include fatty oils such as sesame oil, or synthetic fatty acid esters, such as ethyl oleate or triglyceride, or liposomes. Aqueous injection suspensions may contain substances that increase the viscosity of the suspension, such as sodium carboxymethyl cellulose, sorbitol, or dextran, optionally such as ethyl oleate or triglycerides or liposomes. Aqueous injection suspensions may contain substances that increase the viscosity of the suspension, such as sodium carboxymethyl cellulose, sorbitol, or dextran. Optionally, the suspension may also contain suitable stabilizers or agents that increase the solubility of the compounds to enable the preparation of highly concentrated solutions.

In addition to the preparations described previously, the compounds may also be prepared as a storage preparation. Such long-acting preparations may be administered by implantation (such as subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

For buccal or sublingual administration, the compositions may take the form of tablets, lozenges, pastilles, or gels formulated in a conventional manner. Such compositions may contain the active ingredient in a flavored base such as sucrose and acacia or tragacanth.

The compounds can also be formulated into a composition for rectal administration, such as a suppository or a retention enema, for example, containing a conventional suppository matrix such as cocoa butter, polyethylene glycol, or other glycerol esters.

Certain compounds disclosed herein can be administered topically, i.e. by non-systemic administration. This includes administering the compounds disclosed herein to the eye, epidermis, exterior of the oral chamber, ear, and/or nose, such that the compounds do not significantly enter the bloodstream. In contrast, systemic administration refers to oral, intravenous, intraperitoneal, and intramuscular administration.

The active ingredient for topical administration may account for, for example, 0.001% to 10% w/w (by weight) of the preparation. In certain embodiments, the active ingredient may account for, up to 10% w/w. In other embodiments, it may account for less than 5% w/w. In certain embodiments, active ingredients may account for 2% w/w to 5% w/w. In other embodiments, it may account for from 0.1% to 2% w/w, preferably from 0.1% to 0.5% w/w of the preparation. In certain embodiments, it may account for 0.01% w/w, 0.05% w/w, 0.1% w/w, 0.25% w/w, or 0.5% w/w of the preparation.

In some embodiments, the active ingredient for topical administration may account for, for example, from 0.001% w/v to 10% w/v of the preparation (by weight/volume, unit: g/100 ml). In certain embodiments, the active ingredient may account for up to 10% w/v. In other embodiments, it may account for less than 5% w/v. In certain embodiments, active ingredients can account for 0.2% w/v to 0.5% w/v. In other embodiments, it may account for 0.1% w/v to 2% w/v, preferably 0.1%-0.5% w/v of the preparation. In certain embodiments, it may account for 0.01% w/v, 0.05% w/v, 0.1% w/v, 0.25% w/v, or 0.5% w/v of the preparation.

In a preferred aspect, the preparations for topical administration to the eye or ear in aqueous solution or suspension are in the form of drops. The preparations for topical administration to the nose in aqueous solution or suspension are in the form of drops, sprays or aerosols. The term "aqueous" generally denotes an aqueous preparation in which the preparation contains> 50%, more preferably> 75% and especially> 90% water by weight. These drops may be delivered in a single-dose ampoule, which may preferably be sterile, thus making bacteriostatic components of the preparation unnecessary. Alternatively, the drops may be delivered in a multi-dose bottle which may preferably include a device for withdrawing any preservative therefrom upon delivery of the preparation, such devices being known in the art. Solution and suspension preparation can be administered nasally using an atomizer. Intranasal delivery of solutions, suspensions, or dry powders may also be facilitated by propellant-based aerosol systems, including but not limited to hydrofluorocarbon-based propellants. Alternatively, the active pharmaceutical ingredient may be delivered in the form of a dry powder.

In a specific embodiment, the preparation of this application is administered twice per day. However, the preparation may also be formulated to be administered at any frequency of administration, including once per week, once every 5 days, once every 3 days, once every 2 days, once per day, three times per day, four times per day, five times per day, six times per day, eight times per day, every hour, or at any higher frequency. This frequency of administration is also maintained for varying durations according to the treatment regimen. The duration of a particular treatment regimen may vary from a single administration to a regimen that extending over months or years.

The preparation for topical administration in the mouth, for example buccally or sublingually, includes troches containing the active ingredient in a flavored base such as sucrose and acacia or tragacanth, and lozenges containing the active ingredient in a base such as gelatin and glycerin or sucrose and acacia.

For administration by inhalation, the compound may be conveniently delivered from an insufflator, atomizer pressurized pack, or other convenient means of delivering an aerosol spray. The pressurized pack may contain a suitable propellant, such as hydrofluoroalkane, dichlorodifluoromethane, trichlorofluoromethane, dichlorotetrafluoroethane, carbon dioxide, or other suitable gas. In the case of a pressurized aerosol, the dosage unit may be determined by providing a valve to deliver a metered amount. Alternatively, for administration by inhalation or insufflation, the compound according to the present application may take the form of a dry powder composition, for example, a powder mix of the compound and a suitable powder base such as lactose or starch. The powder composition may be provided in unit dosage form in, for example, a capsule, cartridge, gelatin or blister pack, from which the powder may be administered by means of an inhaler or insufflator.

Preferred preparations in a unit dosage or a single dose are those containing an effective dose, as described below herein, or an appropriate fraction thereof, of the active ingredient.

It should be understood that in addition to the ingredients particularly mentioned above, the preparation described above may also include other reagents in the field that are conventional for the type of preparation of concern, for example, preparations suitable for oral or intranasal administration may include a flavoring agent.

The compound may be administered orally or via injection at a dose of 0.01 to 300 mg/kg per day. The dosage range for adult humans is generally 0.1 mg to 50 mg per day. Tablets or other presentation forms provided in discrete units may conveniently contain an amount of one or more of the compounds that are effective at such dose or doses, for example, 0.05 mg to 100 mg, typically around 1 mg to 50 mg, preferably 5 mg.

The compounds may be administered in various ways, e.g. orally, topically or by injection. The precise amount of compound administered to a patient is the responsibility of the attendant physician. The specific dosage level for any specific patient depends on various factors, including the activity of the specific compound used, age, weight, general health status, gender, diet, administration time, administration route, excretion rate, drug combination, exact symptoms being treated, and the severity of the indications or symptoms being treated. In addition, the route of administration may vary depending on the condition and its severity.

In some cases, it may be appropriate to administer at least one compound described herein (or a pharmaceutically acceptable salt, ester, or prodrug thereof) in combination with another therapeutic agent. By way of example only, if one of the side effects experienced by a patient upon receiving one of the compounds herein is liver injury, it may be appropriate to administer a hepatoprotective drug in combination with the initial therapeutic agent. Alternatively, as an example only, the efficacy of one of the compounds described herein can be enhanced by administering an adjuvant (i.e. the adjuvant itself may only have the minimal therapeutic benefit, but when combined with another therapeutic agent, the overall therapeutic benefit to the patient is enhanced). Alternatively, as an example only, the benefits experienced by patients can be enhanced by administering one of the compounds described herein and another therapeutic agent that also has therapeutic benefits (which also includes a treatment regimen). As an example only in the treatment of myopia involving the administration of one of the compounds described herein, the therapeutic benefit may be enhanced by also providing another myopia treatment agent to the patient, such as atropine. In any case, regardless of the disease, condition, or symptom being treated, the total benefit experienced by the patient can be simply be the sum of two therapeutic agents, or the patient can experience synergistic benefits.

The pharmaceutical compositions in dry or liquid form may be provided as single or multi-dose pharmaceutical compositions.

In one embodiment of the present application, the liquid or dry pharmaceutical composition is provided in a single dose, which means that the container in which it is provided contains one drug dose. Alternatively, the liquid or dry pharmaceutical composition is a multi-dose pharmaceutical composition, which means that the container in which it is provided contains more than one therapeutic dose, i.e. the multi-dose composition contains at least 2 doses. Such multi-dose compositions may be used for different patients in need thereof or may be used for one patient, wherein the remaining doses are stored until needed after application of the first dose.

In another aspect of the application, the pharmaceutical composition is in a container. The container for liquid or dry pharmaceutical composition is, for example, a syringe, a vial, a vial with a plug and a seal, an ampoule, and a cartridge. In particular, the liquid or dry pharmaceutical composition is provided in a syringe. If the pharmaceutical composition is a dry pharmaceutical composition, the container is preferably a dual-chamber syringe. In this embodiment, the dry pharmaceutical composition is provided in a first chamber of a dual-chamber syringe and the reconstituted solution is provided in a second chamber of the dual-chamber syringe.

The dried composition is reconstituted prior to the application of the dried composition to a patient in need thereof. Reconstitution may be performed in a container that provides the dry composition, for example in a vial, syringe, dual-chamber syringe, ampoule, and cartridge. Reconstitution is performed by adding a predetermined amount of reconstitution solution to the dry composition. The reconstitution solution is a sterile liquid such as water or a buffer, which may contain other additives such as a preservative and/or antimicrobial agent such as benzyl alcohol and cresol. Preferably, the reconstitution solution is sterile water. When the dry composition is reconstituted, it is referred to as a "reconstituted pharmaceutical composition" or "reconstituted pharmaceutical composition" or "reconstituted composition".

The pharmaceutical compositions of the present application may be administered in the form of an ophthalmic preparation comprising an ophthalmically acceptable carrier.

The amount of active ingredients in the pharmaceutical composition of the present application. When the drugs are mixed, the concentration of the drugs can be selected to be an effective and suitable amount of each drug.

The preparation and method of the present application have been applied to any subject who may benefit from the preparations and methods of the present application. The subject is typically a mammal, more typically a human. Nevertheless, the present application is not limited to the treatment of humans and may be adapted for veterinary use.

In another aspect, the present application provides a device containing a pharmaceutical composition which including bendazac or a therapeutically acceptable salt thereof (such as bendazac lysine) or a derivative thereof, preferably wherein the device delivers the pharmaceutical composition in a sustained-release manner.

In certain embodiments of the pharmaceutical composition, device, or method of treatment disclosed herein, the device delivers the pharmaceutical composition in a sustained-release manner, preferably, the sustained release has circadian rhythm.

In certain embodiments of the pharmaceutical composition, device, or method of treatment disclosed herein, the pharmaceutical compositions are formulated as ophthalmic compositions, e.g. as ophthalmic compositions for the treatment of ophthalmic disorders or conditions.

In certain embodiments of the pharmaceutical composition, device, or method of treatment disclosed herein, the pharmaceutical composition is formulated as an ophthalmic composition for the treatment of anterior myopia, myopia, or progression of myopia.

In certain embodiments of the pharmaceutical composition, device, or method of treatment disclosed herein, the pharmaceutical composition is formulated as an ophthalmic composition for the treatment of high myopia, moderate myopia, or low myopia.

In certain embodiments of the pharmaceutical composition, device, or method of treatment disclosed herein, the pharmaceutical composition is formulated as an ophthalmic composition for the treatment of a patient diagnosed with (or at risk of developing or predisposed to developing) anterior myopia.

In certain embodiments of the pharmaceutical composition, device, or method of treatment disclosed herein, the pharmaceutical composition is administered ophthalmically to the eye(s) of the patient, preferably, the eye is myopic.

In certain embodiments of the pharmaceutical composition, device, or method of treatment disclosed herein, the pharmaceutical composition is administered topically.

In certain embodiments of the pharmaceutical composition, device, or method of treatment disclosed herein, the pharmaceutical composition is administered ophthalmically to the eye of a patient via a device.

In certain embodiments of the pharmaceutical composition, device, or method of treatment disclosed herein, the pharmaceutical composition is administered 1, 2, 3, 4, or 5 times per day.

Dosage, toxicity, and therapeutic efficacy of therapeutic drugs can be determined by standard pharmaceutical procedures in experimental animals, e.g. for determining the LD50 (lethal dose in 50% of the population) and the ED50 (the dose therapeutically effective in 50% of the population). The dose ratio between toxic and therapeutic effects is the therapeutic index and it can be expressed as the ratio LD50/ED50. Compounds that exhibit high therapeutic indices are preferred. Although compounds with toxic side effects may be used, it should be considered to design a delivery system or suitable mode of administration that targets such compounds to the corresponding tissue or lesion site to minimize potential damage to unrelated cells or tissues and thereby reduce side effects.

The data obtained from the animal studies can be used in formulating a range of dosages for use in humans. The dosage may vary within, and sometimes outside, this range depending upon the type of preparation employed and the route of administration utilized. A series of different doses can be formulated in animal models to obtain indicators including minimum effective concentration, circulating blood drug concentration range after a single administration and multiple administrations, drug exposure, etc. Such results can be used to more accurately help determine useful doses in humans by body surface area conversion.

One skilled in the art will recognize that certain factors may influence the dosage and time for effective treatment of a subject, including, but not limited to, the severity of the disease or disorder, whether the subject is fully cooperating with the treatment, previous treatments, the health conditions and/or age of the subject, and other diseases present. Moreover, the treatment of a subject with a therapeutically effective amount of a therapeutic composition described herein can include a single treatment or a series of treatments.

The use of the method of this application will control, slow down, reduce, delay, and/or slow down the development of myopia in the treated patients within the following range relative to untreated patients: about 5-95%, about 5-90%, about 5-80%, about 5-70%, about 5-60%, about 5-50%, about 5-40%, about 5-30%, about 5-20%, about 10-100%, about 20-90%, about 30-90%, about 40-90%, about 50-90%, or about 75-90%.

Use of a pharmaceutical composition, device, or method of treatment limits the amplitude of diopter becoming negative in the administered subject's eye to about 1.0-6.0 D, 1.0-5.0 D, 1.0-4.0 D, 1.0-3.0 D, 1.0-2.0 D, less than 6.0 D, less than 5.0 D, less than 4.0 D, less than 3.0 D, less than 2.0 D, and less than 1.0 D.

In some embodiments, the methods of the present application are used to halt or reverse the progression of myopia in a treated patient. These patients have high, moderate or low myopia; or the patients are anterior myopia (or at risk of developing myopia).

In some embodiments, the methods of the present application are used to prevent, control, retard, delay, postpone, and/or reduce axial (or longitudinal) growth of the eye of a treated patient.

In some embodiments, the methods of the present application are used to control, retard, reduce, delay, and/or slow (down) the progression of myopia in a patient diagnosed with or at risk of developing myopia, increase the choroidal thickness (ChT) of the eye of the patient (e.g. a myopic eye, an anterior myopic eye, or an eye at risk of developing myopia), and/or decrease the axial (or longitudinal) growth rate of the eye of the patient (e.g. a myopic eye, an anterior myopic eye, or an eye at risk of developing myopia).

In some embodiments, axial (or longitudinal) growth of an eye of a treated patient is controlled, retarded, reduced, delayed, and/or slowed using the method of the present application relative to untreated patient, about 5-95%, about 5-90%, about 5-80%, about 5-70%, about 5-60%, about 5-50%, about 5-40%, about 5-30%, about 5-20%, about 10-100%, about 20-90%, about 30-90%, about 40-90%, about 50-90%, or about 75-90%.

In some embodiments, the application of the method of the present application results in a smaller increase in pupil size or no pupil dilation relative to atropine monotherapy.

In some embodiments, the preparation for oral administration is a solid preparation such as a tablet, capsule, granule, and powder preparation, and liquid preparation such as syrup and beverage preparation; optionally (a): in solid preparations, an excipient, lubricant, binder, disintegrant and the like can be formulated, and preferably, a preservative, antioxidant, colorant and sweetener can be formulated; more preferably, additives are used; optionally (b): in liquid preparations, a solvent, dissolving aid, suspending agent and isotonic agent are included; preferably, an agent, buffer, painless agent and the like can be mixed; more preferably, additives such as a preservative, antioxidant, colorant, sweetener and the like are used.

In some embodiments, the drug can be an injection, tablet, lyophilized powder injection, capsule, effervescent tablet, chewable tablet, buccal tablet (lozenge), granule, ointment, syrup, oral liquid, aerosol, nasal drops, external preparation, oral preparation and the like; preferably the ophthalmic dosage form, including but not limited to eyedrops (eye drops), eye ointment, eye spray, implant, ophthalmic gel, ophthalmic patch, ophthalmic microsphere, ophthalmic sustained-release preparation, periocular injection, or intraocular injection; it can also be a free solution, oil-water mixture, suspension, liniment, lotion, cream, drop, electuary, spray, ointment, patch, paste, pill, suppository, or emulsion.

In some embodiments, an "ophthalmic preparation or drug" and the pharmaceutical composition or preparation of the present application are administered contemporaneously, such as concurrently or sequentially administrated during a particular course of administration (treatment), administrated on the same day, administrated in the same week, administrated in the same month, administrated in the same year; or alternately administrated at intervals, such as alternately administrated at intervals of 4 hours, alternately administrated at intervals of 12 hours, alternately administrated every other day, alternately administrated every other week, alternately administrated every other month and alternately administrated every other year.

The specific examples of this application will be described in detail below. Although the present application has been described in conjunction with these specific embodiments, it will be understood that it is not intended to limit the present application to such specific examples.

### Example 1. Preparation methods and sources of main agents or preparations (pharmaceutical composition)

The bendazac lysine preparation includes two types: commercially available drugs and pharmaceutical compositions formulated by the inventor using only the compound bendazac lysine as an effective pharmaceutical ingredient. Commercially available drugs comprising bendazac lysine (BDL(S)), i.e. 0.5% bendazac lysine eye drops (GYZZ H20063847) commercially available in China, was directly used for topical ocular administration in guinea pig myopia model; the compound bendazac lysine was purchased from MedChemExpress. The compound bendazac lysine as a powder was directly and completely dissolved in 0.9% normal saline to prepare a 5 mg/ml (11.669 mM) preparation (BDL) without adding other pharmaceutical excipients or other compounds. The overall appearance of this preparation at room temperature was clear, transparent, homogeneous, and free from macroscopically visible suspended substances. When administered to a subject, it was directly applied topically to the eye or diluted with 0.9% normal saline according to the dosage requirements before application.

The bendazac preparation includes two types: commercially available drugs that can be directly purchased and pharmaceutical compositions formulated by the inventor using the compound bendazac alone. Among them, the commercially available drug was 3% bendazac ointment purchased from IWAKI SEIYAKU CO., LTD. by the inventor; the compound bendazac was purchased from MedChemExpress. The compound bendazac was completely dissolved in DMSO to prepare a 330 mg/ml (1166.9 mM) stock solution, and stored at -20°C. The stock solution before the start of the experiment was used to prepare working solution in the ratio of stock solution: PEG300: Tween80: 0.9% normal saline = 1: 45: 5: 49, and the final concentration of the resulting bendazac preparation was 3.3 mg/ml (11.669 mM). All preparation operations were performed in a dark room.

Lysine was purchased from MedChemExpress and was completely dissolved in 0.9% normal saline to give a test preparation of 1.7 mg/ml (11.669 mM).

M-hydroxy-methylaniline was purchased from Shanghai Bidepharm Technology Co. Ltd. M-hydroxy-methylaniline was completely dissolved in 0.9% normal saline to prepare a 250 mM stock solution, and stored at -20°C. The m-hydroxy-methylaniline preparation used in the example was diluted with 0.9% normal saline prior to the start of the experiment to a final concentration of 5 mM. All preparation operations were performed in a dark room.

Aldose reductase inhibitor (ARI) Sorbinil, was purchased from MedChemExpress. Sorbinil as a powder was dissolved in DMSO to prepare a 4.8 mg/ml (20 mM) stock solution and stored at -20°C. The stock solution was diluted with 0.9% normal saline prior to the start of the experiment. The final concentration of Sorbinil preparation used in the examples was 24 µg/ml (100 µM), and all operations were performed in a dark room.

Aldose reductase inhibitor Zopolrestat was purchased from MedChemExpress. Zopolrestat as a powder was dissolved in DMSO to prepare an 84 mg/ml (1M) stock solution and stored at -20°C. The stock solution was diluted with normal saline prior to the start of the experiment. The final concentration of Zopolrestat preparation used in the examples was 420 µg/ml (1 mM), and all operations were performed in a dark room.

Atropine as a powder was purchased from Stanford Chemicals, and was dissolved completely in 0.9% normal saline to prepare a 1 mg/ml, i.e., a 0.1% preparation (positive control). All operations were performed in a dark room.

Other preparations or pharmaceutical compositions that were not mentioned in this section were formulated and stored according to conventional laboratory methods and standards. Conventional physical and chemical dissolution assisting means such as heating, stirring and pH adjusting may be used in the preparation process of all preparations as appropriate, and there was no precipitation of the compound in all preparations before administration.

### Example 2. Construction of animal models and experimental methods

The form deprivation myopia and lens induced myopia models are classic and recognized animal models for myopia in this field, which can be used for evaluating the efficacy and safety of myopia treatment drugs. The construction methods are well-known to technical personnel in this field. For both guinea pig models of form deprivation myopia (FDM) and lens induced myopia (LIM), modeling, administration, and data analysis were carried out in accordance with the published literature in the laboratory of the inventor.^{[1-4]} The examples of this application use healthy guinea pigs (i.e. without underlying diseases such as hypertension, hyperglycemia, ocular diseases or abnormalities, etc.) both males and females. The animal experiments described in this application has been reviewed by the Laboratory Animal Ethics Committee of Wenzhou Medical University. The form deprivation and lens induction myopia models of guinea pigs used in Example 3 were constructed as follows:

Tricolor guinea pigs aged 3 weeks were housed in the laboratory animal room of Wenzhou Medical University in a 12-hour light (400-500lux)/12-hour dark environment with free access to water and food. The myopia model was established by monocular form deprivation (FD) and lens induction (LI). The model of form deprivation was performed using a specially designed eye mask that could not fall off itself with a light transmittance of 1% to completely cover the right eye of the animal, while the other eye (left eye) obtained normal vision. Lens induction was performed using -4D lenses fixed in front of the right eye of the animal, and the left eye obtained normal vision. The lens was cleaned twice daily to prevent the lens from blurring. Doses were administered daily at 9: 00 a.m. to 9: 30 a.m. At the time of administration, the eye masks or lenses of the animals in the control and experimental groups were removed under red light, respectively. The right periocular was subconjunctivaly injected with 0.1 ml of the vehicle control or 5 mg/mL of the test drug or positive control. After injection, successful and non-invasive animal administration was confirmed, and the eye mask and lenses were immediately restored. The administration process of each animal was controlled for about 10 seconds. On the day of modeling, animals were administered once daily for 2 weeks for the form deprivation model and once daily for 1 week for the lens induced model.

### Animal grouping

### 1. Form deprivation group:

Form deprivation plus vehicle injection group: a single eye was worn with a eye mask and the worn eye was injected with vehicle normal saline for 2 weeks, sample size = 11.

Form deprivation plus drug injection group: a single eye was worn with a eye mask and the worn eye was injected with bendazac lysine (0.5 mg/day) for 2 weeks, sample size = 14.

Form deprivation plus positive control group: a single eye was worn with a eye mask and the worn eye was injected with 0.1% atropine for 2 weeks, sample size = 12.

### 2. Lens induced group:

Lens induction plus vehicle injection group: a single eye was worn with a -4D lens and the worn eye was injected with vehicle normal saline for 1 week, sample size = 14.

Lens induction plus drug injection group: a single eye was worn with a -4D lens and the worn eye was injected with bendazac lysine (0.5 mg/day) for 1 week, sample size = 14.

### Animal eyeball parameter measurement

All animals in the same model for testing efficacy were measured within the same time period, and the first measurement time was at 3-week-old of the animals. Diopter and axial parameters were measured before the test, at 1-week and 2-week (FD only). The diopter was measured using an eccentric infrared photoretinoscope (EIR) built in the laboratory Each eye was measured 3 times, and the mean value was taken as the final result. Axis parameters of guinea pigs were measured with the A-ultrasonic probe in the CinescanA/B ultrasonic diagnostic instrument (QuantelMedical, Aviso, France). The ultrasonic frequency was 11 MHz, and the ultrasonic propagation speed of different refractive media of the eyeball was set as: anterior chamber 1557.5 m/s, lens 1723.3 m/s, and vitreous 1540 m/s. The measurements included anterior chamber depth (ACD), lens thickness (LT), vitreous chamber depth (VCD), and axial length (AL). About 2 min before the test, 0.5% proparacaine hydrochloride eye drops (Alcon, Belgium) were used to perform ocular surface anesthesia on the test eye of the guinea pig. Each eye was measured 6 times, and the mean value was taken as the final result.

### Example 3. In vivo therapeutic effect experiment of bendazac lysine

Tricolor guinea pigs aged 3 weeks were divided into two groups: form deprivation (FD) and lens induction (LI) groups. The form deprivation group was divided into three subgroups: a negative control group peribulbarly injected with 100 µL of normal saline per day to the molded eyeball, an experimental group peribulbarly injected with 0.5 mg of bendazac lysine in the same volume per day to the molded eyeball, and a positive control group peribulbarly injected with 100 µL of 0.1% atropine per day to the molded eyeball. The lens induction group was divided into two sub-groups: a negative control group peribulbarly injected with 100 µL of normal saline per day to the molded eyeball, and an experimental group peribulbarly injected with 0.5 mg of bendazac lysine in the same volume per day to the molded eyeball. Before the first administration, at 1-week and 2-week of administration (FD only), binocular refraction, vitreous chamber, and axial parameters of all the test animals were measured respectively.

### Experimental results

In FD group, at 1-week of administration, the normal saline injection group induced myopia of -3.75 ± 1.76D, and then induced myopia of -6.17 ± 1.52D at 2-week of administration. Moreover, at 1-week of administration, the bendazac lysine injection group induced myopia of -2.15 ± 1.09D, and inhibited myopia by 42.6% compared with normal saline group; while 0.1% atropine induced myopia of -2.98 ± 1.54D, with a myopia inhibition rate of 20.5%. At 2-week of administration, the bendazac lysine injection group induced myopia of -3.46D, and inhibited myopia by 43.9% compared with a normal saline group; while 0.1% atropine induced myopia of -3.93D, with a myopia inhibition rate of 36.4%. The corresponding increase in vitreous chamber depth and axial length was inhibited by bendazac lysine (see FIG. 1). In conclusion, the bendazac lysine-treated group significantly inhibited the progress of diopter becoming negative, elongated the vitreous chamber depth, and elongated axial length in guinea pigs with form deprivation myopia compared to the normal saline group.

In the LI group, at 1-week of administration, the normal saline injection group induced myopia of -4.42 + 0.95D, while at 1-week of administration, the bendazac lysine injection group induced myopia of -3.49 + 1.15D, with a myopia inhibition rate of 21.0% compared to the normal saline group (FIG. 1). It can be seen that, compared to the normal saline group, the bendazac lysine-treated group significantly inhibited the progress of diopter becoming negative in the guinea pigs with lens induced myopia, and at the same time, the elongation of the vitreous chamber depth and the axial length was inhibited by bendazac lysine.

In addition, no ocular abnormalities and no toxic reactions in individuals were observed in all animals treated with bendazac lysine throughout the experimental period, and relevant indicators of corneal curvature (RCC), anterior chamber depth (ACD), and lens thickness (LT) were also not affected by the test drug (see FIG. 2).

The above experiments showed that bendazac lysine can significantly slow down the progress of diopter becoming negative in individuals with myopia, and also significantly inhibit the axial elongation of the eyes. In particular, bendazac lysine can significantly improve the myopia-related symptoms in guinea pigs with form deprivation and lens induction, suggesting that bendazac lysine can be used in the prevention and control of myopia, especially in the treatment, prevention, or improvement of refractive myopia or axial myopia and related symptoms thereof. The above experimental results of the present application demonstrate that bendazac lysine has a good preventing, slowing, and therapeutic effect on myopia in humans, especially children and adolescents.

### Example 4. Inhibition of bendazac lysine eye drops (0.5%, BDL(S)) as commercial products on the progress of myopia

After excluding individuals with obvious eye diseases or abnormalities, healthy 3-week-old tricolor guinea pigs were tested for diopter (infrared eccentric photometric refractometer) and axis length (A-ultrasonic). Animals with refractive power of 3-8 diopter (D) and binocular anisometropia of not more than 2D were selected and randomly divided into the following 5 groups: form deprivation (FD) + vehicle control (NS, i.e. 0.9% normal saline) group, FD + 0.5% bendazac lysine eye drops (BDL(S)) group, FD + 0.1% atropine, lens induction (LI) + vehicle control (NS) group, LI + 0.5% bendazac lysine (BDL(S)) group. At 8 a.m on the first day of the experiment, guinea pigs were subjected to modeling of form deprivation (FD) or lens induced (LI) myopia. The form deprivation myopia model was made by using a mask method. The headgear was made by the inventor using a 10-inch milky-color non-toxic latex balloon. The guinea pig for modeling was made by covering the headgear on the right eye (experimental eye) and not covering the headgear on the left eye (fellow eye). -4D lenses were used in the lens induced myopia model. The lenses were purchased from Wenzhou Focusee Technology Co. Ltd. The specific parameters were: center degree -4D, base curve 16 mm, and diameter 11.8 mm. The lenses were fixed in front of the right eye (experimental eye) of the guinea pig and the left eye (fellow eye) was left untreated. FD and LI inductions were kept throughout the duration of the bendazac lysine efficacy experiment, and the headgear or lens was removed briefly during dosing or ocular testing (e.g. diopter testing). After the start of the efficacy experiment, the position of the headgear was inspected or the lens was wiped daily at 8: 00 a.m., 12: 00 a.m., and 7: 00 p.m., and before administration, respectively; and the individual whose headgear or lens had fallen off more than 3 times was excluded. Starting from the modeling day, the corresponding vehicle or drug was administered to the experimental eye of FDM at 9:00-10: 00 a.m. via peribulbarly subconjunctival injection in a volume of 100 µl once daily for 2 weeks. The diopter and axis length parameters of test animals were detected at the beginning of this efficacy experiment, 1-week and 2-week of the experiment, respectively. All data collection and processing methods were the same as the literature published by the inventor's laboratory^{[4]}. Statistics were based on the difference between the experimental eye and the fellow eye of the same subject. For the lens induced myopia model, starting from the modeling day, the individual was administered with vehicle (negative control) or drug (bendazac lysine) at 9:00-10: 00 a.m. via peribulbarly subconjunctival injection in a volume of 100 µl once daily for 1 week. At the beginning and end of the experiment, the diopter and axis length parameters of all the test animals were measured, and all the data collection and processing methods were referred to the form deprivation group. All the efficacy experiments above were repeated at least twice. The test drug (0.5% bendazac lysine eye drops, GYZZ H20063847) and vehicle control (negative control group) in this example were produced by the same manufacturer of commercial bendazac lysine eye drop and provided to the inventor.

Results were consistent with the conclusions of Example 3. The changes in diopter and axial length parameters of the animals in the negative control group were consistent with those expected in the myopia model, and the positive control drug atropine showed efficacy in the experiment, which proved that the two myopia models in this experiment were successfully established and could be used for efficacy evaluation of the test drug. The commercially available 0.5% bendazac lysine eye drops in China can significantly inhibit and slow down the progress (development) of myopia in the form deprivation myopia model and the negative lens induced myopia model, i.e. the progress of the diopter becoming negative in individuals with myopia was successfully delayed and effectively controlled by bendazac lysine eye drops, even in some animals in the bendazac lysine treatment group, the inventor found that the progress of myopia was almost completely stopped by the test drug. In terms of diopter of the FDM group, 0.5% bendazac lysine eye drops had better therapeutic effect on myopia than 0.1% atropine eye drops at 1-week after administration, but there was no statistical difference between the two groups; the therapeutic effect of 0.5% bendazac lysine eye drops on myopia was similar to that of 0.1% atropine eye drops at 2-week after administration, and the efficacy of the two groups both had extremely significant difference compared to the negative control group. In the LIM group, bendazac lysine was also effective in inhibiting the progress of myopia. The rate of the diopter becoming negative in the drug intervention group was significantly slower than that in the control group, and there was a significant difference in diopter between the two groups at the end of the experiment, i.e., bendazac lysine was also effective in treating myopia induced by the negative lens. The specific result is that under the same visual information input condition for myopia (both are -4D), the diopter of the drug intervention group was greater than that of vehicle group (both are negative, the mean diopter of the negative control group had reached -4D, and the drug intervention group had not yet) at the same detection time point (at 1-week after administration), indicating that bendazac lysine can effectively control the progress of myopia. At the same time, the increase of vitreous chamber depth (VCD) and the elongation of axial length (AL) of animals treated with bendazac lysine in the above two classic myopia study models were also correspondingly effectively inhibited (there was a statistical difference compared to the negative control group), and the above corresponding indicators at two detection time points in the commercially available bendazac lysine group in the form deprivation test were superior to the atropine group. In addition, no ocular abnormalities and no toxic reactions in individuals were observed in all animals treated with bendazac lysine throughout the experimental period, and relevant indicators of corneal curvature (RCC), anterior chamber depth (ACD), and lens thickness (LT) were also not affected by the test drug. Animals in the positive control group showed pupil dilation after the administration of atropine.

In summary, on the premise that there are no significant adverse drug reactions, the commercial bendazac lysine eye drops can effectively prevent, control, and treat myopia in mammals (including humans), especially slow down and control the progress of myopia, and can significantly improve the relevant symptoms of the myopia disease model. Bendazac lysine can effectively reduce (suppress) the elongation of axis length in an individual with myopia or a tendency to develop myopia, and can effectively reduce (suppress) the increase of vitreous chamber depth, indicating its application in the prevention and control of myopia, whether applied in the treatment, prevention, or improvement of refractive myopia or axial myopia and related symptoms thereof. Therefore, the commercial bendazac lysine eye drops can be used for the treatment of myopia (nearsightedness), especially to control the progress rate of the disease, so as to reduce the complications and the risk of blindness caused by high myopia, and thus it can be used for the intervention treatment in children and adolescents.

### Example 5. Test of dose-response relationship of bendazac lysine at different concentrations (0.01%, 0.05%, 0.1%, and 0.5%) for the treatment of myopia.

After excluding individuals with obvious eye diseases or abnormalities, healthy 3-week-old tricolor guinea pigs were tested for diopter (infrared eccentric photometric refractometer) and axis length (A-ultrasonic). Animals with refractive power of 3-8 diopter (D) and binocular anisometropia of not more than 2D were selected and randomly divided into the following 6 groups: FD + normal saline group (negative control), FD+0.01% bendazac lysine, FD+0.05% bendazac lysine, FD+0.1% bendazac lysine, FD+0.5% bendazac lysine (experimental group, meaning applying 0.5% bendazac lysine to individuals in FDM model), FD+0.1% atropine (positive control). To be more intuitive, percentage are converted to milligram units when plotting. At 8 a.m on the first day of the experiment, the guinea pigs were subjected to modeling of form deprivation (FD) myopia. The form deprivation myopia model was made by using a mask method. The headgear was made by the inventor using a 10-inch milky-color non-toxic latex balloon. The guinea pig for modeling was made by covering the headgear on the right eye (experimental eye) and not covering the headgear on the left eye (fellow eye). FD induction was kept throughout the duration of the bendazac lysine efficacy experiment, and the headgear was removed briefly only during dosing or ocular testing (e.g. diopter testing). After the start of the efficacy experiment, the position of the headgear was inspected daily at 8: 00 a.m., 12: 00 a.m., and 7: 00 p.m., and before administration, respectively; and the individual whose headgear had fallen off more than 3 times was excluded. Starting from the modeling day, the corresponding vehicle (negative control) or drug was administered to the experimental eye of the FDM at 9:00-10: 00 a.m. via peribulbarly subconjunctival injection in a volume of 100 µl once daily for 2 weeks. The diopter and axis length parameters of test animals were detected at the beginning of the efficacy experiment, 1-week and 2-week of the experiment, respectively. All data collection and processing methods were the same as the literature published by the inventor's laboratory^{[4]}. Statistics were based on the difference between the experimental eye and the fellow eye of the same subject. All the efficacy experiments above were repeated at least three times, and the bendazac lysine preparation was self- formulated by the inventor using normal saline.

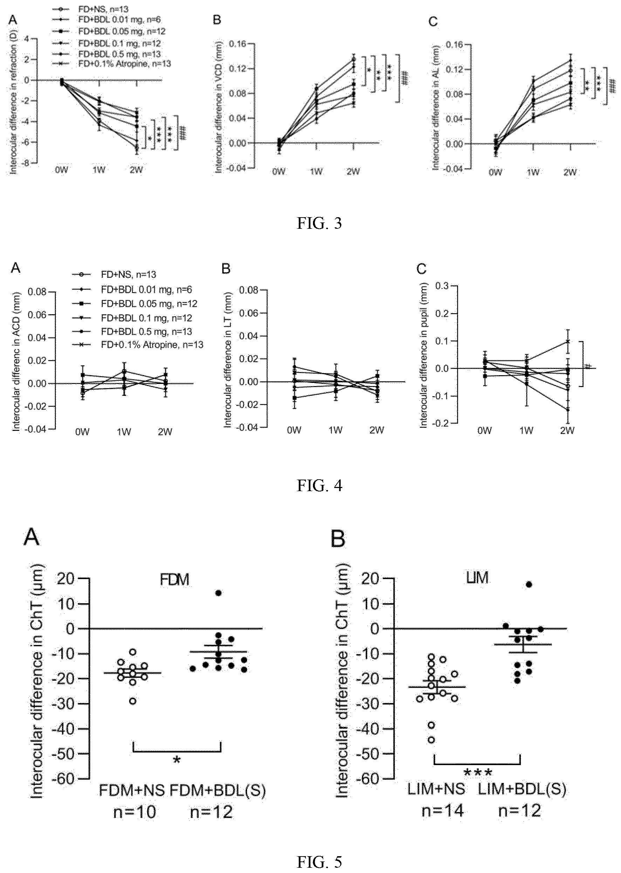

The experimental results showed that the changes in diopter and axial length parameters of the animals in the negative control group were consistent with those expected in the myopia model, and the positive control drug atropine showed efficacy in the experiment, which proved that the myopia model was successfully established in this experiment, and could be used to evaluate the efficacy of bendazac lysine. Compared with the negative control group, bendazac lysine (without other pharmaceutical excipients except for 0.9% normal saline as the vehicle) dose-dependently retarded the progress of myopia in subjects. With the increase of the administration dose of bendazac lysine, the inhibition rate of myopia with diopter as a parameter (the calculation formula is: (diopter of drug group - diopter of vehicle group)/diopter of vehicle group) also increased accordingly, indicating that the more bendazac lysine is administered, the better effect for preventing and controlling myopia (nearsightness) is. In terms of the parameter diopter, the efficacy of the bendazac lysine preparation formulated by the inventor using only normal saline was consistent with that of the commercially available bendazac lysine drug, i.e. 0.5% bendazac lysine had better therapeutic effect on myopia than 0.1% atropine at 1-week after administration, but there was no statistical difference between atropine and bendazac lysine; the therapeutic effect of 0.5% bendazac lysine on myopia was similar to that of 0.1% atropine eye drops at 2-week after treatment. The two groups both had extremely significant difference in the efficacy compared to the negative control group. This indicates that bendazac lysine as the main active ingredient or the only active ingredient can play a therapeutic and preventive effect on myopia. Statistically, the 0.05% and 0.1% bendazac lysine preparations also showed a significant therapeutic effect on myopia (effectively inhibiting the progress of diopter becoming negative). In the low-concentration group, 0.01% bendazac lysine was tested to be similar to the negative control group in terms of the parameter diopter at 1-week, and slightly stronger than the negative control group at 2-week, but there was no statistical difference. 0.05%, 0.1%, and 0.5% of bendazac lysine can also inhibit the increase in vitreous chamber depth (VCD) and the elongation of axial length (AL) in individuals with myopia. Among them, in addition to the experimental group of 0.01% bendazac lysine as the lowest concentration, other higher concentrations of bendazac lysine, like atropine, can effectively inhibit the elongation of the posterior segment (vitreous chamber depth and axial length) of the eye of the individuals and can effectively control, inhibit, delay or slow down the (continuous) progress of diopter becoming negative in individual with myopia or a tendency to develop myopia. Compared with the vehicle group, the above-mentioned inhibition effect has a significant difference or extremely significant difference (see FIG. 3). No ocular irritation and any ocular abnormalities were observed in all the experimental groups of bendazac lysine at all concentrations during administration. Relevant indicators such as anterior chamber depth, lens thickness, and pupil were not affected by the administration of bendazac lysine. Pupil dilation was observed in all the animals in the positive control atropine group after administration, with a statistical difference compared to the vehicle group (see FIG. 4), which was consistent with the adverse drug reactions reported in the clinical trial.

The results of this example demonstrated that the use of bendazac lysine alone may prevent and treat myopia (nearsightedness), or bendazac lysine may be used to prepare preparations or pharmaceutical compositions that may be used for prevention and treatment of myopia (nearsightedness). Preferably, the concentration of bendazac lysine in the above preparation or pharmaceutical composition is not less than 0.01% and may range from 0.05% to 1%. Considering that the FDA (Food and Drug Administration) believes that bendazac may cause severe hepatotoxicity in human body and the drugs for treating myopia should be continuously administered for a long time, as well as the special requirements of regulatory authorities on the safety of pediatric drugs, the inventors believe that bendazac lysine eye drops with a concentration of 0.1 %-0.25% is a reasonable formula for clinical prevention and control of myopia, namely, the concentration of bendazac lysine in the preparation or pharmaceutical composition is preferably 0.1%-0.25%. At the same time, in addition to increasing the concentration of bendazac lysine when preparing a bendazac lysine preparation, the ways to achieve or improve the prevention and control effect of bendazac lysine on myopia in the population with myopia or a tendency to develop myopia include increasing the ocular bioavailability of bendazac lysine, increasing the administration frequency of drugs, using in combination with other drugs for treating myopia, and optimizing the formulation of bendazac lysine preparation, etc. In summary, the bendazac lysine preparations or pharmaceutical compositions described above are effective in reducing (inhibiting) the elongation of the axial length of the eye in the individual(s) with myopia or a tendency to develop myopia and in reducing (inhibiting) the increase of the vitreous chamber depth in the individual(s) with myopia or a tendency to develop myopia; the bendazac lysine preparation or pharmaceutical composition is capable of treating (preventing and controlling) myopia, especially for the treatment of myopia in people aged 6 to 18 years.

### Example 6. Bendazac lysine can significantly increase the choroidal thickness

### (I) Bendazac lysine effectively inhibit the decrease of choroidal thickness in FDM and LIM models of guinea pigs

After excluding individuals with obvious eye diseases or abnormalities, 3-week-old tricolor guinea pigs were tested for diopter (infrared eccentric photometric refractometer) and axis length (A-ultrasonic). Animals with refractive power of 3-8 diopter (D) and binocular anisometropia of not more than 2D were selected and randomly divided into the following 4 groups: form deprivation (FD) + vehicle control (NS) group, FD + 0.5% bendazac lysine eye drops (BDL(S)) group, lens induction (LI) + vehicle control (NS) group, LI + 0.5% bendazac lysine (BDL(S)) group. At 8 a.m. on the first day of the experiment, guinea pigs were subjected to modeling of form deprivation (FD) or lens induced (LI) myopia. The form deprivation myopia model was made by using a mask method. The headgear was made by the inventor using a 10-inch milky-color non-toxic latex balloon. The guinea pig for modeling was made by covering the headgear on the right eye (experimental eye) and not covering the headgear on the left eye (fellow eye). -4D lenses were used in the lens induced myopia model. The lenses were purchased from Wenzhou Focusee Technology Co. Ltd. The specific parameters were: center degree -4D, base curve 16 mm, and diameter 11.8 mm. The lenses were fixed in front of the right eye (experimental eye) of the guinea pig and the left eye (fellow eye) was left untreated. FD and LI induction were kept throughout the duration of the bendazac lysine efficacy experiment, and the headgear or lens was removed briefly only during dosing or choroid thickness measurements. After the start of the efficacy experiment, the position of the headgear was inspected or the lens was wiped daily at 6: 00 a.m., 12: 00 a.m., 6: 00 p.m., and before administration, respectively, and the individual whose headgear or lens had fallen off more than 3 times was excluded. Starting from the modeling day, the corresponding vehicle or drug was administered to the experimental eye of the FDM at 9:00-10: 00 a.m. via peribulbarly subconjunctival injection of the experimental eye in a volume of 100 µl once daily for 2 weeks. For the lens induced myopia model, starting from the modeling day, the individual was administered with vehicle (negative control) or drug (bendazac lysine) via parabulbarly subconjunctival injection of the experimental eye at 9:00-10: 00 a.m. once daily for 1 week. The choroidal thickness of all eyes in the two myopia models was detected by Spectralis HRA + OCT (Heidelberg Engineering, Heidelberg, Germany) at 30-60 min after the last administration. All the data were collected and processed in the same manner as the literature published by the inventor's laboratory^{[4]}. The statistical basis was the difference between the experimental eye and the fellow eye of the same subject. The statistical method was an independent sample T-test. All the efficacy experiments above were repeated at least twice. The test drug (0.5% bendazac lysine eye drops) and vehicle control (negative control group) in this example were produced by the same manufacturer of commercial bendazac lysine eye drop and provided to the inventor.

Results showed that, in both FDM and LIM models, bendazac lysine can effectively increase the choroidal thickness of guinea pigs in both FDM and LIM models compared with the vehicle control group, and there was a statistical difference between the two groups (the difference between the vehicle control group and drug group in LIM model was extremely significant). The specific efficacy manifestation was that choroidal thickness in the experimental eyes of guinea pigs in the vehicle control group in both the FDM and LIM model was decreased, while bendazac lysine inhibited the decrease of choroidal thickness in eyes with myopia. In the FDM model, the mean difference in choroidal thickness between the experimental eye and the fellow eye was -17.51 µm, while the mean difference of choroidal thickness between the experimental eye and the fellow eye was -9.20 µm in the individuals with myopia after the intervention of bendazac lysine, indicating that the choroidal thickness of the experimental eye was close to that of the normal eye in the same subject after the drug intervention (the difference between the two was decreased); in the LIM model, the mean difference in choroidal thickness between the experimental and the fellow eyes was -23.38 µm, while in some individuals with myopia, the mean difference in choroidal thickness between the experimental and the fellow eyes was -6.31 µm after the bendazac lysine intervention, even in some individual animals, the choroidal thickness of the experimental eye completely recovered to normal eye level after administration (see FIG. 5). The indicators corneal curvature (RCC), anterior chamber depth (ACD), and lens thickness (LT) were also unaffected by the test drug. In conclusion, bendazac lysine can significantly inhibit the decrease of choroidal thickness and slow down the decrease of choroidal thickness in individuals with myopia or a tendency to develop myopia.

In individuals with myopia, after the parallel rays have been refracted by adjusting the refractive system of the relaxed eye, a focus falls in front of the retina. As bendazac lysine increases the thickness of the choroid in individuals with myopia, it moves the retina towards the lens, resulting in a shortened or even coincidental distance between the imaging focus of myopic eyes and the retina. The distance between the imaging focus and the retina, i.e. the degree of myopia, can be reduced or inhibited by bendazac lysine in individuals with myopia, which in turn reduces the degree of myopia. Administration of bendazac lysine to a individual with myopia results in a better-defined distance vision of the treated eye and an effective reduction in the myopic refractive state (myopia being treated to reduce the degree of myopia), including an improvement in the distance vision. Therefore, the treatment (prevention and control) effect produced by bendazac lysine on myopia in the present application is not only limited to axial myopia, refractive myopia, pathological myopia, simple myopia, pseudo-myopia or true myopia but is also unrelated to factors such as age, gender, degree of myopia, speed of progress of myopia, nationality and age at which myopia occurs of the subject to be treated, i.e., bendazac lysine has a treatment and prevention and control effect on all types of myopia.

### Example 7. Therapeutic effects of eyedrop administration of bendazac lysine eye drops and eye smear administration of bendazac ointment (Noninvasive or Non-Invasive administration)

After excluding individuals with obvious eye diseases or abnormalities, 3-week-old tricolor guinea pigs were tested for diopter (infrared eccentric photometric refractometer) and axis length (A-ultrasonic). Animals with refractive power of 3-8 diopter (D) and binocular anisometropia of not more than 2D were selected and randomly divided into three groups: FD + normal saline group, FD + 0.5% bendazac lysine eye drops (self-made), FD + 0.1% atropine. At 8: 00 a.m on the first day of the experiment, the guinea pigs were subjected to modeling of form deprivation., using the mask method, with the right eye (experimental eye) covered and the left eye (fellow eye) uncovered. FD induction was kept throughout the duration of the bendazac lysine efficacy experiment, and the headgear was removed briefly only during dosing or ocular testing (e.g. diopter testing). After the start of the efficacy experiment, the position of the headgear was inspected daily at 8: 00 a.m., 12: 00 a.m., and 7: 00 p.m., and before administration, respectively; and the individual whose headgear had fallen off more than 3 times was excluded. Starting from the modeling day, the corresponding vehicle or drug was administered to the experimental eye at 9:00-10: 00 a.m. daily for direct eye drop administration, and the eye drop was administered at 2: 30 to 3: 30 p.m. i.e. twice per day, with a volume of 25 µl each time daily, for consecutive 2 weeks. The diopter and axis length parameters of test animals were detected at the beginning of the efficacy experiment, 1-week and 2-week of the experiment, respectively. All data collection and processing methods were the same as the literature published by the inventor's laboratory^{[4]}. Statistics were based on the difference between the experimental eye and the fellow eye of the same subject. All efficacy experiments above were repeated at least twice.

Because of the modeling approach, the guinea pig eye structure (eyeballs are relatively convex to humans and do not actively close the eye), and the normal blinking of the animal, the effectively therapeutic dose of the drug actually obtained in each treated eye is lower than the dose of the eye administered by peribulbar injection in the same volume, and the total volume of the test animal daily by eye drop administration is also lower than the dose volume of Example 5 by injection administration. Therefore, in this example, the effect of both bendazac lysine eye drops and 0.1% atropine administered by direct eye drop administration is inferior to that for treating myopia by peribulbar injection administration. The changes in diopter and axial length parameters of the animals in the negative control group were consistent with those expected in the myopia model, and the positive control drug atropine showed efficacy in the experiment, which proved that the myopia model was successfully established in this experiment, and could be used to evaluate the efficacy of bendazac lysine. In terms of axial length parameters, both bendazac lysine and 0.1% atropine can significantly inhibit elongation of axial length in the form deprivation myopia model compared with the normal saline group (there was a statistical difference), and both showed almost the same inhibition effect on axial length increase and vitreous chamber depth change in individuals with myopia. Compared with 0.1% atropine eye drop group, the inhibition rates of bendazac lysine eye drop on myopia at 1-week and 2-week were close (35.5% VS. 28.1% and 33.9% VS. 36.9%, respectively, and both had statistical differences in the efficacy compared to the negative control vehicle group) (see FIG. 6). However, the atropine group produced pupil dilation adverse reactions during the administration, while the bendazac lysine group produced no obvious ocular abnormalities. The relevant indicators of corneal curvature, anterior chamber, and lens in subjects were not affected in either atropine or bendazac lysine as intervention on myopia (see FIG. 7). In the laboratory of the inventor, 0.01% atropine eye drops were commonly used as the current clinical trial concentration. Under the same administration conditions in this example (myopia model, administration mode, administration frequency, and administration volume were consistent). No therapeutic effect was observed in the guinea pig in myopia model at this concentration of atropine, regardless of diopter or axial length parameter as the indicator(s), which may be related to the short residence time of eye drops on the surface of the eyeball after administration of the eye drops to animals. Therefore, from the perspective of efficacy and safety, bendazac lysine is superior to atropine in the risk-benefit ratio for the treatment of myopia (e.g. bendazac lysine used during the day will not cause photophobia similar to that caused by mydriasis produced by atropine during the treatment of myopia), especially for the treatment of myopia in children and adolescents and prevention and control of myopia in the school-age population.

For ointment dosage form of the test drug, no control group was set in this example because the 3% bendazac ointment used was commercially available in Japan, the inventor could not obtain the corresponding preparation without bendazac, and atropine ointment containing the same excipients as the bendazac ointment was excluded for the same reason. However, with regard to the efficacy evaluation of the dosage form of bendazac for the treatment of myopia, the inventor can still refer to the indicators in the same batch of the same model control group in the non-invasive administration experiment in the present application. The specific experimental procedures included: the treated eye (experimental eyes) of the guinea pig in the deprivation myopia model described in the present application was applied with bendazac ointment on the corneal surface and periocular skin, and the eyelids were quickly closed 10 times by hand, but the inventor found that a part of bendazac ointment was residued on the inner surface of the eye mask when the next administration was performed because the ointment could not be quickly absorbed and was covered by the eye mask and the animals blinked normally. Bendazac ointment was administered at a dose of 18 mg ± 2 mg per dose. Starting from the modeling day, the drug was administered to the deprived eyes at 9:00-10: 00 a.m. of the day, and the drug was administered for the second time at 2: 30-3: 30 p.m., i.e. twice per day for consecutive 2 weeks. The diopter and axis length parameters of test animals were detected at the beginning of the efficacy test in this model, 1-week and 2-week of the experiment, respectively. All data collection and processing methods were the same as the literature published by the inventor's laboratory^{[4]}. Statistics were based on the difference between the experimental eye and the fellow eye of the same subj ect.

The inhibition rate of myopia was 17.7% and 25.9% after 1-week and 2-week administration of 3% bendazac ointment, which was inferior to 0.5% bendazac lysine or 0.1% atropine in the same batch, but much better than the untreated group in the same batch in the form deprivation myopia model. It is possible that the modeling mask affected the ocular absorption of bendazac in the ointment, or the bioavailability of the bendazac drug in the eyes of guinea pigs proved that it was not a reasonable formulation because the dosage form was mainly targeted to the human skin administration route. However, bendazac (ointment) still showed effective prevention and control of myopia. In addition to inhibiting the progress of diopter becoming negative in individuals with myopia, the increase of vitreous chamber depth and axial length in individuals with myopia was correspondingly inhibited after administration. Specifically, at 2-week after administration, the mean increase of vitreous chamber depth and axial length in the myopia treatment group with bendazac ointment was 0.09 mm and 0.09 mm, respectively, and the corresponding indicators in the non-intervention group in the same batch of myopia were 0.13 mm and 0.12 mm (see FIG. 6). It can be seen that the elongation of the axial length in individuals with myopia is mainly caused by the increase in vitreous chamber depth, and bendazac can simultaneously reduce the increase in vitreous chamber depth and the elongation of the axial length in individuals with myopia. After administration of bendazac, the animals showed no obvious ocular abnormalities, and the relevant indicators of pupil, corneal curvature, anterior chamber, and lens in the subjects were not affected by the drug (see FIG. 7).

In summary, in addition to ocular injection, bendazac and its salt forms (e.g. lysine salts) are also effective in the treatment and prevention of myopia using other noninvasive (non-invasive) modes of administration, and in delaying the progress of diopter becoming negative in an individual with myopia or a tendency to develop myopia. In particular, direct eye drop administration of bendazac lysine eye drops or eye ointment administration of bendazac eye drops is used to treat myopia, inhibit elongation of axial length, and reduce the increase in vitreous chamber depth in the individual with myopia or a tendency to develop myopia.

### Example 8. Effect of simple lysine and simple bendazac eye drops on myopia

After excluding individuals with obvious eye diseases or abnormalities, 3-week-old tricolor guinea pigs were tested for diopter (infrared eccentric photometric refractometer) and axis length (A-ultrasonic). Animals with refractive power of 3-8 diopter (D) and binocular anisometropia of not more than 2D were selected and randomly divided into the following 5 groups: FD + normal saline group (NS), FD + lysine (L-lysine), FD + 0.5% bendazac lysine (BDL), FD + DMSO (vehicle control group set for bendazac, vehicle), FD + bendazac (experimental group, refers to applying bendazac to individuals in FDM model). At 8: 00 a.m. on the first day of the experiment, the guinea pigs were subjected to modeling of form deprivation., using the mask method, with the right eye (experimental eye) covered and the left eye(fellow eye) uncovered. FD induction was kept throughout the duration of the efficacy experiment, and the headgear was removed briefly only during dosing or ocular testing (e.g. diopter testing). After the start of the efficacy experiment, the position of the headgear was inspected daily at 8: 00 a.m., 12: 00 a.m., and 7: 00 p.m., and before administration, respectively; and the individual whose headgear had fallen off more than 3 times was excluded. Starting from the modeling day, the corresponding vehicle or drug was administered to the experimental eye at 9:00-10: 00 a.m., via subconjunctival injection in a volume of 100 µl, once daily for 2 weeks. The diopter and axis length parameters were measured at the beginning of the efficacy experiment, 1-week and 2-week of the experiment, respectively. All data collection and processing methods were the same as the literature published by the inventor's laboratory^{[4]}. Statistics were based on the difference between the experimental eye and the fellow eye of the same subject. All the efficacy experiments above were repeated at least three times, and all the pharmaceutical preparations were self-formulated by the inventor.

The experimental results showed that the changes in diopter and axial length parameters of the animals in both the normal saline and DMSO vehicle negative control groups were consistent with those expected in the myopia model, and 0.5% bendazac lysine as the positive control showed efficacy in the experiment, which proved that the myopia model was successfully established in this experiment, and could be used for the efficacy evaluation of bendazac and lysine. Simply using the same molar amount of lysine as that in 0.5% bendazac lysine had no inhibitory effect on the progress of myopia, regardless of the progress of diopter becoming negative or the elongation of vitreous chamber depth and axial length. For the diopter at 1-week and 2-week during the administration process, the degree of myopia in the lysine intervention group was even higher than that in the normal saline group, while the same molar amount of bendazac lysine effectively inhibited the progress of diopter becoming negative in the administered individuals and there was a statistical difference between the two groups (see FIG. 8). Therefore, lysine does not have any therapeutic or prophylactic effect on myopia, bendazac lysine exerts its therapeutic effect on myopia without a direct causal relationship with the lysine component in its molecule, and any pharmaceutically acceptable salt of bendazac can be used for the treatment and prevention of myopia.

Compared with its DMSO vehicle control group, the myopia inhibition rate at 1-week and 2-week with bendazac alone was 33.4% and 30.1%, which was consistent with that of the same batch of equimolar bendazac lysine positive control group, and there was a statistical difference between the two groups and their corresponding negative controls. It was proved that bendazac was the key and only part for the effect of bendazac lysine molecule in the treatment of myopia, and it could effectively inhibit and slow down the progress of diopter becoming negative in individuals with myopia. The administration of bendazac also significantly inhibited elongation of axial length and reduced the increase of vitreous chamber depth in individuals with myopia compared with the DMSO vehicle group, and there was a statistical difference compared to the negative control group, with a similar effect to that of the same batch of equimolar bendazac lysine experimental group (see FIG. 9).

After administration of bendazac lysine, bendazac, or lysine, the animals showed no obvious ocular abnormalities, and the relevant indicators of pupil, corneal curvature, anterior chamber depth, and lens thickness of the subjects were not affected by the drug (see FIGs. 10 and 11).

In the above-mentioned experiment, the choroidal thickness was measured at 2-week after administration of bendazac and its corresponding vehicle negative control according to the protocol described in the present application. The results showed that bendazac alone (in the form of eye drops) could inhibit the reduction of the choroidal thickness in myopic eyes. The mean difference in the choroidal thickness between the experimental eyes and the fellow eyes in individuals with myopia was -18.82 µm, while the mean difference in the choroidal thickness between the experimental eyes and the fellow eyes in individuals with myopia after bendazac intervention was -8.47 µm. There was a statistical difference between the bendazac drug group and the DMSO vehicle group (see FIG. 12). Thus, bendazac and bendazac lysine show an efficacy consistency in inhibiting the decrease in choroidal thickness in individuals with myopia or a tendency to develop myopia, and bendazac can significantly inhibit the decrease in choroidal thickness and slow down the decrease in choroidal thickness in individuals with myopia or a tendency to develop myopia.

The above results demonstrate that, bendazac and its corresponding compounds in any of its salt forms (e.g. bendazac lysine) are effective in the treatment of myopia by inhibiting the elongation of axial length and slowing the increase in the vitreous chamber depth to retard the progress of diopter becoming negative in individuals with myopia or a tendency to develop myopia. Also, bendazac and pharmaceutically acceptable salts thereof (e.g. bendazac lysine) are effective in increasing choroidal thickness and reducing degree of myopia. The dosage forms of bendazac and its pharmaceutically acceptable salts (e.g. bendazac lysine) in the treatment and prevention of myopia can be eye drops, eye ointments, eye sprays, eye injections, and eye gels. Devices, preparations, or pharmaceutical compositions containing such compounds (drugs) can be used to control the progress of myopia.

### Example 9. The therapeutic effect of Sorbinil and Zopolrestat on guinea pig in form deprivation myopia model

After excluding individuals with obvious eye diseases or abnormalities, 3-week-old tricolor guinea pigs were tested for diopter (infrared eccentric photometric refractometer) and axis length (A-ultrasonic). Animals with a refractive power of 3-8 diopter (D) and binocular anisometropia of not more than 2D were selected and randomly divided into three groups: FD+DMSO, FD+Sorbinil, FD+Zopolrestat. According to the compound database information, IC₅₀ of Zopolrestat = 3.1 nM, IC₅₀ of sorbinil = 3.14 ± 0.02 µM, the final concentration of Zopolrestat preparation actually used in the inventor's experiment was 1 mM, and the final concentration of Sorbinil preparation was 100 µM. At 8 a.m. on the first day of the experiment, guinea pigs were subjected to modeling of form deprivation myopia. The form deprivation myopia model was made by using a mask method. The headgear was made by the inventor using a 10-inch milky-color non-toxic latex balloon. The guinea pig for modeling was made by covering the headgear on the right eye (experimental eye) and not covering the headgear on front of the left eye (fellow eye). FD induction was kept throughout the duration of the bendazac lysine efficacy experiment, and the headgear was removed briefly only during dosing or ocular testing (e.g. diopter testing). After the start of the efficacy experiment, the position of the headgear was inspected daily at 8: 00 a.m., 12: 00 a.m., and 7: 00 p.m., and before administration, respectively; and the individual whose headgear had fallen off more than 3 times was excluded. Starting from the modeling day, the corresponding vehicle or drug was administered to the experimental eye of the FDM at 9:00-10: 00 a.m. via parabulbar subconjunctival injection in a volume of 100 µl once daily for 2 weeks. The diopter and axis length parameters of test animals were detected at the beginning of the efficacy experiment, 1-week and 2-week of the experiment, respectively. All data collection and processing methods were the same as the literature published by the inventor's laboratory^{[4]}. Statistics were based on the difference between the experimental eye and the fellow eye of the same subject. All the efficacy experiments above were repeated at least 3 times, and the test drug and vehicle control (negative control group) in this example were self-formulated by the inventor.

The results showed that Sorbinil and Zopolrestat had no therapeutic effect on myopia and could not control the progress of myopia. These two aldose reductase inhibitors cannot inhibit the progress of diopter becoming negative induced by FD and slow the progress of elongation of axial length in individuals with myopia. There were no significant difference between both drugs, Sorbinil and Zopolrestat, and the vehicle group in either diopter or axial length parameter as the indicator at each test time point throughout the experimental period (FIG. 13). In addition, the administration of Sorbinil and Zopolrestat had no effect on the relevant indicator of the corneal curvature, anterior chamber depth and lens thickness of the subjects (see FIG. 14). In summary, aldose reductase is not a target for the development of drugs for the treatment of myopia, and not all aldose reductase inhibitors have the effect of preventing and controling myopia.

### Example 10. The therapeutic effect of m-hydroxy methylaniline on guinea pig in form deprivation myopia model

The efficacy was evaluated using the guinea pig in form deprivation myopia model described in this application. All subjects were measured for diopter and axial length. After excluding the non-eligible animals, they were randomly divided into 3 groups: FD + 0.9% normal saline (NS), FD + m-hydroxy methylaniline (Compound A), and FD + 0.1% atropine (positive control). At 8: 00 a.m. on the first day of the experiment, the guinea pigs were subjected to modeling of form deprivation, using the mask method, with the right eye (experimental eye) covered and the left eye (fellow eye) uncovered. Starting from the modeling day, the corresponding group of experimental eyes was given the vehicle or drug at 9:00-10: 00 a.m. daily via parabulbar subconjunctival injection in a volume of 100 µl, once daily for 1 week. Diopter and axis length parameters were measured at the beginning and after the end of the efficacy experiment. All the data collection and processing methods were the same as those in other examples of the present application, and the statistics were based on the difference between the experimental eye and the fellow eye of the same subject.

The changes in diopter and axial length parameters of the animals in the negative control group in this study were consistent with those expected in the myopia model, and the positive control drug atropine showed efficacy in the study, which proved that the myopia model in this study was successfully established and could be used for the efficacy evaluation of the test drug. Experimental results are shown in FIG. 15. Neither the progress of diopter becoming negative nor the increase in vitreous chamber depth or axial length of the eye was inhibited after administration of m-hydroxy methylaniline. It indicates that m-hydroxy methylaniline has no effect of preventing or treating myopia. Neither the diopter nor axial length parameters in individuals showed statistical differences compared to the negative control group after administration of m-hydroxy methylaniline, indicating that this drug had no inhibitory effect on elongation of axial length and no slowing effect on the increase of vitreous chamber depth in individuals with myopia. Atropine exhibited normal effect for treating myopia in this example, but all animals tested in this group showed pupil dilation, and anterior chamber depth and lens thickness were unaffected by the drug intervention (FIG. 15). In summary, the experimental results demonstrate that: not all compounds (drugs) with effect for treating cataract can be used to treat myopia; not all compounds with an activity for antioxidant or reducing BLOA (Biological Liquid Oxidant Activity) possess effect for preventing and controlling myopia.

Finally, it should be noted that the above embodiments are only used to illustrate the technical solutions of this application, which are not to limited by them. Although the present application has been described in detail with reference to the aforementioned embodiments, those of ordinary skill in the art should understand that they can still modify the technical solutions described in the aforementioned embodiments, or equivalently substitute some or all of the technical features. These modifications or substitutions do not make the spirit of the corresponding technical solutions deviate from the scope of the technical solutions of the various embodiments of this application, and they should all be covered within the scope of the claims and specifications of this application. In particular, the technical features mentioned in the various embodiments can be combined in any manner as long as there is no structural conflict. This application is not limited to the particular embodiments disclosed herein but includes all embodiments falling within the scope of the claims.

### References

1 Lu F, Zhou X, Zhao H, et al. Axial myopia induced by a monocularly-deprived facemask in guinea pigs: A non-invasive and effective model. Exp Eye Res 2006;82:628-636.
2 Lu F, Zhou X, Jiang L, et al. Axial myopia induced by hyperopic defocus in guinea pigs: A detailed assessment on susceptibility and recovery. Exp Eye Res 2009;89: 101-108.
3 Wu H, Chen W, Zhao F, et al. Scleral hypoxia is a target for myopia control. Proc Natl Acad Sci U S A 2018;115:E7091-E7100.
4 Pan M, Zhao F, Xie B, et al. Dietary omega-3 polyunsaturated fatty acids are protective for myopia. Proc Natl Acad Sci U S A 2021;118.

## Claims

1. Use of bendazac lysine or bendazac, or an optical isomer thereof, a racemate thereof, a solvate thereof, a pharmaceutically acceptable salt thereof, a prodrug thereof, a metabolite thereof, an analog thereof or a derivative thereof, a crystalline compound thereof, or a combination of these substances, in one or at least two of the following:
(a) preventing and/or treating myopia and myopia-related symptoms;
(b) delaying, reducing, or treating an abnormal development of eyeball associated with ametropia;
(c) enabling an individual to obtain clearer distance vision without using or replacing lenses (such as myopia frame glasses or OK lenses) or relying on other vision correction means (such as refractive surgery);
(d) controlling, inhibiting, delaying, or slowing down the progress (speed) of diopter (continuously) becoming negative in an individual with myopia or a tendency to develop myopia;
(e) preventing and/or treating myopia and related symptoms thereof in combination with surgery (such as refractive correction surgery, myopia corneal laser surgery, lens surgery) or other vision correction means (such as corneal contact lenses);
(f) preventing and/or treating myopia and myopia-related symptoms in combination with one or more other drugs;
(g) reducing a distance between a retina and a lens, preferably reducing the distance between the retina and the lens in an individual with myopia or a tendency to develop myopia;
(h) reducing a degree of myopia, or treating myopia, or treating nearsightedness, or controlling a progression of myopia, or correcting myopia, or alleviating myopia, or preventing myopia in adolescents;
(i) inhibiting or treating myopia caused by lens lesions; and
(j) manufacturing a pharmaceutical composition, preparation, or device to fulfill at least one of the uses of (a) to (i) above.

2. Use according to the preceding claims, wherein bendazac lysine or bendazac, or the optical isomer thereof, the racemate thereof, the solvate thereof, the pharmaceutically acceptable salt thereof, the prodrug thereof, the metabolite thereof, the analog thereof or the derivative thereof, the crystalline compound thereof, or the combination of these substances acts as a sole active ingredient or as a main active ingredient.

3. The use according to claim 2, wherein a content of the sole active ingredient or the main active ingredient accounts for 50% or more, 60% or more, 70% or more, 80% or more, 90% or more, or 100% of total active ingredients, in which the percentage is a mass ratio or a molar ratio.

4. The use according to any one of the preceding claims, wherein these substances or the combination thereof and the one or more other drugs are formulated or designed in a manner of continuous administration, simultaneous administration, sequential administration, alternating administration, interval administration, or separate administration.

5. The use according to any one of the preceding claims, wherein the abnormal development of eyeball associated with ametropia is developed during the juvenile phase of an individual (such as 2-28 years of age in humans), and is mainly induced by environmental factors or mainly caused by human factors (such as long-term near-distance reading, frequent use of electronic screens, persistent nearsightedness and lack of chance for farsightedness, improper use of refractive corrective glasses, side effects of drugs, obesity, trauma, poor lighting in the learning environment, lack of outdoor exercises), while genetic factors are secondary factors, concomitant factors, synergistic factors, or the abnormal development is a refractive dysplasia completely independent of genetic factors, mainly **characterized in that** in a state of accommodative relaxation, a focus falls in front of the retina after parallel light rays are refracted through a refractive system of eyes.

6. The use according to any one of the preceding claims, wherein systemic administration (such as oral administration, intravenous drip), topical administration (such as eye drop, intravitreal injection, skin cream or ointment application), parenteral administration (such as via mucosal administration, transdermal administration, microneedle administration), non-invasive administration (such as by applying an ophthalmic ointment to the cornea or squeezing into a sac formed by stretching of the lower eyelid), or non-invasive administration (such as using an ophthalmic spray) is used.

7. The use according to claim 6, wherein the skin cream or ointment application is a application with 3% skin cream or ophthalmic ointment, in which the percentage (%) being expressed as mass/volume concentration (ratio) or mass ratio or molar (number) ratio.

8. The use according to any one of the preceding claims, wherein the administrations (such as eye drop, oral administration) are used simultaneously, in combination, alternately, at intervals, separately, or one of them selected for use.

9. The use according to any one of the preceding claims, wherein a preparation for topical administration comprises but is not limited to aqueous, oily, or suspension preparation, that can incorporate pharmacologically and/or physiologically active ingredients such as a mydriatic component, decongestant component, eye muscle (such as ciliary muscle) regulating component, anti-inflammatory component, astringent component, antihistamine component, antiallergic component, hepatoprotective (avoiding or attenuating hepatotoxicity) component, blood-retinal barrier enhancing component (making it more difficult for compounds to penetrate through the physiological barrier), vitamin, amino acid, antibacterial agent component, saccharide, polymer or derivative thereof, cellulose or derivative thereof, local anesthetic component, glaucoma treatment component, cataract treatment component, and the like.

10. The use according to any one of the preceding claims, wherein a concentration or a ratio of these substances or the combination thereof in the pharmaceutical composition, preparation, or device is at least not less than 0.01%, preferably 0.01% to 0.8%, preferably 0.05% to 0.5%, more preferably 0.1%; or the concentration or the ratio of these substances or the combination thereof is less than 0.01%, in which the percentage being expressed as mass/volume concentration (ratio) or mass ratio or molar (number) ratio.

11. The use according to any one of the preceding claims, wherein the pharmaceutical composition or preparation can be an injection, tablet, lyophilized powder injection, capsule, effervescent tablet, chewable tablet, buccal tablet (lozenge), granule, ointment, syrup, oral liquid, aerosol, nasal drops, external preparation, oral preparation and the like; preferably ophthalmic dosage form, including but not limited to eyedrops (eye drops), eye ointment, eye spray, implant, ophthalmic gel, ophthalmic patch, ophthalmic microsphere, ophthalmic sustained-release preparation, periocular injection or intraocular injection; or a free solution, oil-water mixture, suspension, liniment, lotion, cream, drop, electuary, spray, ointment, patch, paste, pill, suppository or emulsion.

12. The use according to any one of the preceding claims, wherein the individual with myopia or a tendency to develop myopia is a human being, and can be a child, adolescent, middle-aged person or elderly person, preferably a person aged 3 to 26, more preferably a person aged 6 to 18; or an adult or minor, preferably those whose eyes (eyeballs) are still in the growth and development stage; or a school-age people, preferably students in grades 1 to 12.

13. The use according to any one of the preceding claims, wherein the myopia is refractive myopia or axial myopia; congenital myopia (birth or preschool myopia), early-onset myopia (under 14 years old), delayed myopia (16 to 18 years old), late-onset myopia (after adulthood); low myopia (mild myopia), moderate myopia, high myopia (severe myopia); pseudo-myopia, true-myopia, semi-true and semi-pseudo (mixed) myopia; myopia in children and/or adolescents (preferably the age of the population is 3 to 26, more preferably the age of the population is 6 to 18), myopia in minors, myopia in adolescents, myopia in adults, myopia in the elderly; simple myopia, pathological myopia; axial simple myopia, simple axial myopia; axial myopia in children and/or adolescents (preferably the age of the population is 3 to 26, more preferably the age of the population is 6 to 18); axial myopia in school age and pre-school population; primary myopia, secondary myopia; primary myopia in children and/or adolescents (preferably the age of the population is 3 to 26, more preferably the age of the population is 6 to 18); progressive myopia in children and/or adolescents (preferably the age of the population is 3 to 26, more preferably the age of the population is 6 to 18); curvature myopia, index myopia, positional myopia, bending myopia; myopia caused by long-term short-distance use of eye(s), myopia and pseudo-myopia caused by asthenopia, negative diopter caused by adverse drug reactions, nearsightedness, myopia caused by reading, myopia caused by the use of electronic products such as mobile phones, myopia caused by mismatch of refractive media (components), refractive myopia, myopia caused by abnormal refractive development, myopia caused by excessive eyeball growth, myopia caused by unhygienic use of eyes, myopia caused by imaging focus of distant object falling in front of the retina caused by various reasons, myopia caused by poor or ineffective treatment of atropine, myopia caused by insufficient outdoor exercises, and accommodative tension myopia, childhood myopia, or myopia dominated by environmental factors.

14. Use according to any one of the preceding claims, wherein the myopia-related symptoms comprise complications resulting from myopia, such as complications resulting from high myopia, muscae volitantes, glaucoma, posterior scleral staphyloma, retinal detachment, retinal tear, amblyopia, macular hemorrhage, choroidal neovascularization, choroidal atrophy, macular degeneration or maculopathy, visual field loss, progressive or sudden decrease in vision (especially near vision), ocular soreness and/or pain, night blindness, astigmatism, anisometropia, blindness, vitreous liquefaction, vitreous opacity, strabismus, frequent blinking, frequent eye rubbing, anisometropia, blurred vision when looking at distant objects, squinting or partially closing eyelids to see distant objects clearly, headache caused by asthenopia, difficulty in vision when driving, especially at night (night myopia), retinal atrophy and degeneration (bleeding and tears), subretinal neovascularization, and eyeball atrophy.

15. The use according to any one of the preceding claims, wherein the pharmaceutical composition, preparation, or device further comprises a medicinal preparation or drug comprising, but not limited to, a drug for treating myopia (e.g. atropine, dibazole, pirenzepine, muscarinic antagonist, 7-methylxanthine (7MX), aminobenzylamine, indoleamine, timolol maleate, epinephrine, pirenzepine, pyrazine, pybenpine, pibenpine, pyenzepine, methylamine, chlorisondamine, acetylcholinesterase inhibitor, dopamine agonist, gamma-aminobutyric acid, naloxone, glucagon, retinoic acid, etc.), M receptor blocker (such as a blocker or antagonist or inhibitor for the M3 receptor), bendazac or various salt forms thereof, bendazac lysine or various salt forms thereof, polyunsaturated fatty acid (such as DHA, EPA), salidroside, formononetin, prazosin, homatropine, anisodamine (racemic), tropicamide, nicotinic acid, piracetam, salvia miltiorrhiza extract, safflower extract, fish oil, bear bile extract, vitamin, ATP, non-selective adenylate antagonist, vasodilator, mydriatic, smooth muscle relaxant, vasospasm preventing drug, collagen metabolism regulating drug, anti-allergic drug, anti-inflammatory drug, liver-protecting drug, therapeutic component for ophthalmic disease, topical ophthalmic anesthetic, or ophthalmic preparation.

16. The use according to any one of the preceding claims, wherein the one or more other drugs comprise, but are not limited to, a drug for treating myopia (e.g. atropine, dibazole, pirenzepine, muscarinic antagonist, 7-methylxanthine (7MX), pirenzepine, aminobenzylamine, indoleamine, timolol maleate, epinephrine, pyrazine, pybenpine, pibenpine pyenzepine, methylamine, chlorisondamine, acetylcholinesterase inhibitor, dopamine agonist, gamma-aminobutyric acid, naloxone, glucagon, retinoic acid, etc.), M receptor blocker (such as a blocker or antagonist or inhibitor for the M3 receptor), bendazac or various salt forms thereof, bendazac lysine or various salt forms thereof, polyunsaturated fatty acid (such as DHA, EPA), salidroside, formononetin, prazosin, homatropine, anisodamine (racemic), tropicamide, nicotinic acid, piracetam, salvia miltiorrhiza extract, safflower extract, fish oil, bear bile extract, vitamin, ATP, non-selective adenylate antagonist, vasodilator, smooth muscle relaxant, vasospasm preventing drug, collagen metabolism regulating drug, anti-allergic drug, anti-inflammatory drug, liver-protecting drug, therapeutic component for ophthalmic disease, topical ophthalmic anesthetic, mydriatic, or ophthalmic preparation or drug.

17. The use according to any one of the preceding claims, wherein the preparation can also be an oral product such as a health product, food product, dietary supplement, nutritional product, drink, or a cosmetic product; wherein the cosmetic product can be one or more of a free solution, oil-water mixture, suspension, liniment, lotion, spray, cream, drop, electuary, ointment, paste, pill, suppository, emulsion, and patch.

18. The use according to any one of the preceding claims, wherein the device is an instrument, apparatus, consumable, system, medical device, health care product or product for changing the appearance of the eye, capable of releasing a drug or having a drug delivery function or a potential drug delivery capability, such as corneal contact lens(es), glasses, intraocular lens, suture, OK lens(es) cleaning (maintenance) system, eye patch, eyesight improving patch, cosmetic lens(es), microneedle, eye spray system, eye massager (myopia massager), eye fumigator, ocular surface drug delivery device, intraocular drug delivery device, fundus drug delivery device, implanted pump, wearable equipment, acupoint massage instrument, eye relaxation equipment, myopia treatment instrument, or drug-device combination for myopia prevention and control.
